# EUROPÄISCHE PATENTSCHRIFT

(11) **EP 0 912 897 B1**
(45) Veröffentlichungstag und Bekanntmachung des Hinweises auf die Patenterteilung: **28.08.2002**
(21) Anmeldenummer: 97923094.3
(22) Anmeldetag: 16.05.1997
(51) Int. Cl.: G01N 33/543, C12M 1/40, C12Q 1/00

(54) **ENZYMATISCH-ELEKTROCHEMISCHER EINSCHRITTAFFINITÄTSSENSOR ZUR QUANTITATIVEN BESTIMMUNG VON ANALYTEN IN WÄSSRIGEN MEDIEN UND AFFINITÄTSASSAY**
ENZYMATIC-ELECTROCHEMICAL ONE-SHOT AFFINITY SENSOR FOR THE QUANTITATIVE DETERMINATION OF ANALYTES IN AQUEOUS MEDIA AND AFFINITY ASSAY
DETECTEUR D'AFFINITE A UN SEUL CYCLE DE TRAITEMENT ENZYMATICO-ELECTROCHIMIQUE POUR DETERMINATION QUANTITATIVE DE LA PRESENCE D'ANALYTES DANS DES SUBSTANCES AQUEUSES ET ANALYSE D'AFFINITE

(30) Priorität: 24.05.1996 DE 19622458
(43) Veröffentlichungstag der Anmeldung: 06.05.1999
(73) Patentinhaber: Senslab Gesellschaft zur Entwicklung und Herstellung Bioelektrochemischer Sensoren mbH, 04347 Leipzig (DE); UFZ-UMWELTFORSCHUNGSZENTRUM LEIPZIG-HALLE GMBH, 04318 Leipzig (DE)
(72) Erfinder: GRÜNDIG, Bernd, D-04318 Leipzig (DE); RENNEBERG, Ilka, D-13187 Berlin (DE); STREHLITZ, Beate, D-04277 Leipzig (DE); KOPINKE, Holm, D-04357 Leipzig (DE)
(74) Vertreter: Ziebig, Marlene, Dr. Dipl.-Chem.
(86) Internationale Anmeldenummer: EP9702518
(87) Internationale Veröffentlichungsnummer: WO97045738

(56) Entgegenhaltungen:
- EP-A- 0 223 541
- WO-A-86/03837
- DE-A- 2 642 321
- US-A- 4 145 255
- US-A- 4 376 825
- ANALYTICAL BIOCHEMISTRY, Bd. 199, Nr. 2, Dezember 1991, NEW YORK, NY, Seiten 269-274, XP002040555 BAYMANN ET AL.: "An electrochemical assay for the characterization of redox proteins from biological electron transfer chains"

## Beschreibung

Die Erfindung betrifft einen enzymatisch-elektrochemischen Affinitätssensor und einen Einschritt-Affinitätsassay zur quantitativen Bestimmung von Analyten in wäßrigen Medien. Die Erfindung bezieht sich insbesondere auf ein enzymatisch-elektrochemisches Signalamplifizierungssystem zur hochsensitiven Indikation von Affinitätsreaktionen und ist vor allem in Form eines Einschritt-Affinitätssensors zur Vor-Ort-Analytik geeignet. Gegenstand der Erfindung ist auch die Verwendung von Phenoloxidase als Markerenzym für die affinen Bindungspartner in einem elektrochemischen Affinitätssensor bzw. -assay sowie die Verwendung eines phenolische Verbindungen hydrolysierenden Enzyms als Markerenzym für die affinen Bindungspartner in Kombination mit einer Phenoloxidase als Katalysator für die Amplifizierungsreaktion in einem elektrochemischen Affinitätsassay.

Zum Nachweis immunochemischer Reaktionen bzw. generell von Affinitätsreaktionen sind eine Reihe elektrochemischer bzw. enzymatisch-elektrochemischer Indikationssysteme bekannt, die auf der Indikation eines elektrochemisch aktiven Markers (Heinemann & Halsall, *Anal. Chem., 57 (1985), pp. 1321A-1331A;* Patentschrift DE 4216696; Le Gal La Salle, *J. Electroanal. Chem., 350 (1993), 329-335)* oder elektrochemisch aktiven Produkts des Markerenzyms beruhen. Als Markerenzym dient vor allem alkalische Phosphatase *(Doyle et al., Anal.Chem. 56 (1984), pp.2355-2360;* McNeil, et al., *Biosensors 3 (1987), pp. 199-209;* Duan et. al., *Anal. Chem. 66 (1995), pp. 1369-1377;* Meusel et al. *Biosens. & Bioelectron. 10 (1995), pp. 577-586*) oder Galactosidase (Màsson et al., *Anal. Chim. Acta, 304 (1995), pp. 353-359).* Eine Vielzahl anderer enzymatisch-elektrochemischer Indikationssysteme basiert auf der zyklischen Regenerierung redoxaktiver Reaktionspartner.

Am häufigsten beschrieben sind dabei Assays, die entweder ein Redoxenzym oder einen Redoxmediator als Marker für ein Antigen oder einen Antikörper verwenden. Nach entsprechender immunochemischer Reaktion wird durch den Marker in Anwesenheit des Enzymsubstrats eine enzymatische Redoxreaktionssequenz komplettiert, bei der ein Redoxmediator, eine redoxaktive prosthetische Gruppe des Enzyms oder ein redoxaktiver enzymatischer Cofaktor bzw. ein redoxaktives Cosubstrat reduziert oder oxidiert wird. Mittels einer amperometrischen Redoxelektrode wird das Redoxsystem direkt oder via Mediator regeneriert. Der daraus resultierende Strom ist abhängig von der Analytkonzentration. D.h., das analytabhängig aus der Immunoreaktion nur in geringer Konzentration verfügbare redoxaktive Konjugat verursacht eine - zyklische enzymatisch-elektrochemische Regenerierungsreaktion, die eine amplifizierende Signalbildung mit einem entsprechend hohen Meßstrom zur Folge hat. Der Redoxmarker kann Bestandteil eines homogenen oder eines heterogenen Immunoassays bzw. eines Immunoassays nach dem Konkurrenz-, Titrations- oder Verdrängungsprinzip sein.

Durch Weber und Purdy (*Anal. Letters 12(1979), pp.1-9*) wurde erstmals ein homogenes Immunoassay unter Verwendung von Ferrocen als redoxaktives Label eines Antigens realisiert und die direkte Oxidation des Ferrocenkonjugats bei -500 mV (vs. SCE) detektiert.
In einem enzymatisch-elektrochemischen Immunoassay (diGleria et al., *Anal. Chem. 58(1986), pp.1203-2206*) wird ebenfalls Ferrocen als Antigenlabel verwendet, wobei Glucoseoxidase zur mediierten Indikatorreaktion des Antigen-Ferrocenkonjugats dient, das bei Anwesenheit von Analyten aus der Bindungsstelle des Antikörpers verdrängt wird und damit den Elektronentransfer zwischen der Oxidase und der Elektrode übernehmen kann.

Die Vervollkommnung dieses enzymatisch-elektrochemischen Immunoassays wird in der Patentschrift *EP 125 139* beschrieben. Als Label für das Antigen bzw. den Antikörper dient wieder ein Redoxmediator. In der Publikation von Suzawa et.al. *(Anal. Chem., 66 (1994), pp. 3889-3894)* wird Ferrocen als Mehrfachlabel in Kombination mit Glucoseoxidase genutzt. Ein anderes bekanntes Immunoassay (Gyss and Bourdillon, *Anal.Chem., 59 (1987), pp.2350-2355*) verwendet Glucoseoxidase als Marker, wobei als Mediator Benzochinon dient.

Die Steigerung der Empfindlichkeit amperometrischer Indikationssysteme auf der Basis von Redoxenzym-Mediator-Sequenzen in Immunoassays hat die Patentschrift *EP 241 309* zum Ziel. Dabei wird ein zweiter Elektronenakzeptor (Ferricyanid, Polyvinylferrocen oder Berliner Blau) in die Meßlösung eingebracht bzw. zur Modifizierung der Elektrodenoberflache verwendet und via Hapten-Ferrocenkonjugat zur Akkumulierung von Reduktionsäquivalenten aus der enzymatischen Glucoseoxidation verwendet. Nach einer Anreicherungszeit erfolgt die amperometrische Messung des in dieser Anreicherungsphase reduzierten Elektronenakzeptors.

In der Patentschrift *EP 223 541* werden Redoxmediatoren verwendet, die durch das Ankoppeln einer Phosphatgruppe bzw. eines Phenolderivats in ihrem formalen Potential in negative Richtung verschoben sind, so daß im Unterschied zu ihrer nicht derivatisierten Form kein Elektronentransfer von Glucoseoxidase via Mediator auf die Elektrodenoberfläche erfolgen kann. Als Mediator dient Ferrocen oder Dichlorphenolindophenol. In Anwesenheit eines Haptenkonjugats, das alkalische Phosphatase als Markerenzym aufweist und aus einer Konkurrenzreaktion mit dem Analyten resultiert, erfolgt eine Abspaltung der derivatisierenden Gruppe, so daß der Mediator die Elektronenübertragung zwischen Glucoseoxidase und der Redoxelektrode übernehmen kann. In Abhängigkeit von der Konzentration des verfügbaren Markerenzym-Antigen/ Antikörperkonjugats tritt ein anodischer Meßstrom auf.

Weiterhin ist ein Meßsystem bekannt (*PCT 86*/*03837*), bei dem das Markerenzym selbst nicht die indizierende Redoxreaktion katalysiert, sondern eine redoxaktive "Triggersubstanz" erzeugt; letztere ist entweder direkt amperometrisch detektierbar oder komplettiert aufgrund ihres reversiblen Redoxverhaltens eine enzymatisch-elektrochemische Signalamplifizierungssequenz. Als Markerenzym dient alkalische Phosphatase oder β-Galactosidase. Im Fall der alkalisehen Phosphatase wird als "Triggersubstrat" NADP⁺ verwendet, das durch Abspaltung einer Phosphatgruppe zu NAD⁺ hydrolysiert wird und als Cofaktor in einer Ethanol/Alkoholdehydrogenase/Diaphorase-, Ethanol/Alkoholdehydrogenase/ Ferricyanid- oder Ethanol/Alkoholdehydrogenase/Ferrocen/Ferricyanid-Redoxelektroden-Sequenz zur Vervollständigung dieser Redoxkaskade führt. In analoger Weise sind auf der Basis der direkten oder mediatorgekoppelten Indikation des Cofaktors NADH elektrochemisch-enzymatische Assays von Eggers et. al. (*Clin. Chem., 28*/*9 (1982), pp. 1848-1851*) und Cardosi et. al. (*Electroanalysis, 1 (1989), 297-304*) beschrieben. Alternativ kann das Markerenzym alkalische Phosphatase zur Abspaltung der Phosphatgruppe einer SH-haltigen Verbindung dienen, so daß diese als Elektronendonator für eine Glutathionreduktase oder Diaphorase fungiert und kathodisch regeneriert wird. Außerdem wird in dieser Patentschrift die Verwendung von β-Galactosidase als Markerenzym beschrieben, das p-Hydroxyphenyl-β-galactosid hydrolysiert. Das gebildete p-Hydroxychinon dient als Substrat für die Laccase und wird wiederum kathodisch regeneriert, so daß der resultierende Reduktionsstrom proportional zur Konzentration des Antikörper/Enzymkonjugats ist.
Aus der Patentschrift *PCT 86*/*04926* ist ein enzymatischelektrochemisches Indikationssystem für immunochemische Reaktionen bekannt, das räumlich voneinander durch ein Polymer getrennt ein Depolymerase/Ligandkonjugat und eine Redoxsequenz, bestehend aus Oxidoreduktase, Mediator und Redoxelektrode aufweist, oder das eine der genannten Redoxkomponenten polymergebunden bzw. in ein Polymer eingeschlossen enthält. Als Polymer wird vorzugsweise ein Polysaccharid oder eine Liposomenmatrix, als Depolymerase Amyloglucosidase, α-Amylase oder Phospholipase, als Mediator Ferrocen oder ein Ferrocenderivat und als Redoxenzym Glucoseoxidase oder Glucosedehydrogenase verwendet.
Durch die als Markerenzym genutzte Depolymerase werden von einem Polymer Monomere abgespalten, die als Substrat für eine Oxidoreduktase dienen oder als mediatorgekoppelte Monomere diffusionsfähig werden, so daß in beiden Fällen eine enzymatisch-elektrochemische Reaktion generiert wird. Andere mögliche Depolymerasereaktionen bewirken das Freisetzen des in Liposomen eingeschlossenen Redoxenzyms oder die Penetrierung einer Polymermembran, die zuvor den Mediator vom Redoxenzym getrennt hat. Der aus der Redoxsequenz resultierende amperometrische Strom ist wiederum proportional zur Markerenzym-Ligandkonjugatkonzentration.

Schließlich werden Peroxidase-gekoppelte elektrochemisch-enzymatische Immunoassays beschrieben. In einem bekannten homogenen elektrochemisch-enzymatischen Immunoassay (*J.P. O' Daly et al., Enzyme Microb. Technol., 14 (1992) 4, pp. 299-302*), dient ein Ferrocenderivat als Label für ein Hapten, das durch Bindung des Konjugats an den Antikörper zunächst elektrochemisch inaktiv ist. Bei Anwesenheit des Analyten erfolgt eine Verdrängungsreaktion und der als Haptenlabel verwendete Mediator vervollständigt eine Peroxidase-katalysierte Redoxreaktion, die einen der Analytkonzentration proportionalen Reduktionsstrom generiert. In umgekehrter Arbeitsweise (*Pritchard et. al., Anal. Chim. Acta, 310 (1995) pp.251-256)* wurde Peroxidase zum Markieren von Antikörpern verwendet, wobei nach einem Mehrschrittverfahren der Label via Ferrocenmonocarbonsäure kathodisch detektiert wird. Für einen Einwegimmunosensor, der in einem kompetitiven Mehrschritt-Assay dem Nachweis eines niedermolekularen Analyts dient (*Kàlab und Sklàdar, Anal Chim Acta, 304 (1995), pp. 361-368*), wurde Peroxidase als Enzymlabel des Antikörperkonjugats verwendet, das nach kompetitiver Reaktion innerhalb einer definierten Inkubationszeit zwischen dem Analyten und dem Hapten, das an der Elektrodenoberfläche immobilisiert war, und nach einem Spülschritt mittels Hydrochinon an der Elektrode nachgewiesen wurde. In einer anderen bekannten Arbeit (*Wright et al., Biosens. & Bioelectron., 10 (1995), pp. 495-500*) wird Glucoseoxidase als Enzymlabel verwendet, dessen Reaktionsprodukt (Wasserstoffperoxid) bei der eigentlichen Indikationsreaktion über eine mediatorfreie im Siebdruck hergestellte Peroxidase-Kohlenstoffelektrode erfolgt. Diese bekannten elektrochemisch-enzymatischen Indikationssysteme immunochemischer Reaktionen bzw. von Affinitätsreaktionen weisen jedoch den allgemeinen Nachteil auf, daß sie entweder zu aufwendig oder zu unempfindlich für reale Applikationen sind.

Der Erfindung lag deshalb die Aufgabe zugrunde, ein Affinitätsassay zu schaffen, das sich durch eine hohe Empfindlichkeit, einfache Handhabung und eine elektrische -Quantifizierung des Meßsignals auszeichnet. Es soll insbesondere in Form eines Einschritt-Affinitätssensors zur Vor-Ort-Analytik in wäßrigen Medien geeignet sein.

Erfindungsgemäß wird die Aufgabe mittels eines enzymatisch-elektrochemischen Einschritt-Affinitätssensors gemäß Anspruch 1 und der bevorzugten Ausführungsvarianten der Unteransprüche 2 bis 18 sowie mittels eines Affinitätsassays gemäß Anspruch 19 und der dazugehörigen Ausführungsvarianten der Unteransprüche 20 bis 25 gelöst.
Insbesondere ist der erfindungsgemäße Sensor bzw. Assay zum Nachweis niedermolekularer Substanzen wie Pestiziden, PAKs, PCBs, Chlorphenolen, Schwermetallionen, Pharmaka und hochmolekularen Substanzen wie Peptiden, Hormonen, Proteinen, Nukleinsäuren, Glykoproteinen, Zellbotenstoffen sowie zum Nachweis von Mikroorganismen und Viren geeignet.
Die Erfindung ist also insbesondere auf den Gebieten der Umweltanalytik, der medizinischen Diagnostik, Lebensmittelanalytik und zur Kontrolle biotechnologischer Prozesse einsetzbar.

Im Sinne der Erfindung bedeuten Ligand oder Rezeptor die affinen Bindungspartner, zum Beispiel im Falle eines Immunoassays Antigen und Antikörper. Die enzymmarkierten Bindungspartner werden als Konjugate bezeichnet, die Enzyme als Markerenzyme. Die Komplexe zwischen Ligand und Rezeptor werden als Affinitätskomplexe bezeichnet bzw. als komplexierte Konjugate, wenn einer der affinen Bindungspartner des Komplexes enzymmarkiert ist.

Das der Erfindung zugrundeliegende signalamplifizierende enzymatisch-elektrochemische System zum Nachweis von Affinitätsreaktionen beruht auf der Verwendung entweder eines mit Phenoloxidase markierten Liganden, der mit Rezeptoren eine affine Reaktion eingeht, oder eines mit Phenoloxidase markierten Rezeptors, der mit modifiziertem Liganden oder dem Analyten eine affine Reaktion eingeht. Das in der kompetitiven Reaktion nicht an einen immobilisierten Rezeptor oder Liganden gebundene, oder während der kompetitiven Reaktion verdrängte Phenoloxidase-Rezeptorkonjugat oder Phenoloxidase-Ligandenkonjugat oder der nach einer pseudohomogenen Bindungsreaktion zwischen Analyt und Phenoloxidase-Rezeptorkonjugat gebildete Komplex diffundiert durch nacheinander angeordnete Festphasen, die aus diffusionsfähigem oder immobilisiertem Phenoloxidase-Ligandkonjugat oder diffusionsfähigem oder immobilisiertem Phenoloxidase-Rezeptorkonjugat und darauffolgenden Diffusionsbarrieren bestehen, zu einer räumlich entfernten Redoxelektrode (3). In unmittelbarer Oberflächennähe wird ein vorgespeichertes Enzymsubstrat der Phenoloxidase durch die Phenoloxidase des Konjugats bzw. des komplexierten Konjugats zu einem elektroaktiven Produkt oxidiert, das via reversibel reduziertem Elektronenmediator katodisch zu einem Ausgangssubstrat der Phenoloxidase reduziert wird. Die resultierende zyklische Substratregenerierung führt zu einer chemischen, mittels voltammetrischer Methoden quantifizierbaren analytproportionalen Stromsignalverstärkung. Bei einem in analoger Weise als Markerenzym verwendeten hydrolysierenden Enzym, vorzugsweise einer Phosphatase oder Galactosidase, wird in unmittelbarer Elektrodenoberflächennähe Phenoloxidase als Schicht 21 fixiert und ein Edukt, das aus der Hydrolyse eines in Schicht 20 vorgespeicherten Hydrolasesubstrats resultiert, durch die als katalytische Schicht genutzte Phenoloxidase zu einem elektroaktiven Produkt oxidiert und wiederum via reversibel reduziertem Elektronenmediator kathodisch zu einem Ausgangssubstrat der Phenoloxidase reduziert. Die resultierende zyklische Substratregenerierung führt zu einer chemischen, mittels voltammetrischer Methoden quantifizierbaren, analytproportionalen Stromsignalverstärkung.

Als Rezeptor werden im Sinne der Erfindung monoklonale oder polyklonale Antikörper, Antikörperfragmente, Lektine, Protein A und G, Nucleinsäuren, biologische Rezeptoren oder Gemische davon verwendet. Als Ligand gegen den Rezeptor dienen affine Haptene, Peptide, Schwermetallionenkomplexe, Nucleinsäuren, Kohlenhydrate, Proteine, Mikroorganismen, Viren oder Teile von Mikroorganismen oder Viren.

Als Phenoloxidase wird vorzugsweise Tyrosinase eingesetzt. Als hydrolisierendes Enzym ist alkalische Phosphatase besonders bevorzugt. Geeignete Enzymsubstrate der Phenoloxidase, die zu einer starken Signalbildung führen, sind vorzugsweise Phenol, m-Kresol, p-Kresol, 2,4-Xylenol, p-Chlorphenol oder Catechol.

Als Elektronenmediator in der enzymatisch elektrochemischen Indikationsreaktion dient ein chinoider Redoxfarbstoff, ein Chinon, redoxaktive Komplexverbindungen des Eisens, Rutheniums, Osmiums, Cobalts oder Wolframs, ein Metallocen, Phthalocyanin oder ein elektrisch leitfähiges Redoxpolymer wie Polyanilin, Polypyrrol, Poly-o-phenylendiamin oder Polyacetylen.

Eine erste erfindungsgemäße Ausführungsform nutzt das Verdrängungsprinzip. Es beruht auf der Verwendung eines entweder Phenoloxidase-Ligandkonjugats oder Phenoloxidase-Rezeptorkonjugats, das zum Absättigen der Bindungsstellen eines entsprechenden immobilisierten Rezeptors oder immobilisierten Liganden dient. Dabei wird entweder die Phenoloxidase mit einem Liganden konjugiert, der im Vergleich zum eigentlichen Analyten eine wesentlich geringere Affinität gegen den Rezeptor aufweist, oder es wird ein Ligand zur Immobilisierung verwendet, zu dem der Phenoloxidase-markierte Rezeptor eine im Vergleich zum eigentlichen Analyten wesentlich geringere Affinität aufweist Als Ligand dient ein dem Analyten strukturell verwandtes Molekül. Bei Anwesenheit von Analyt erfolgt durch diesen eine Verdrängung des Phenoloxidase-Ligandkonjugats von den Bindungsstellen des immobilisierten Rezeptors oder eine Verdrängung des immobilisierten Liganden durch den Analyten von den Bindungsstellen des mit Phenoloxidase konjugierten Rezeptors. Das verdrängte Phenoloxidase-Ligandkonjugat bzw. das mit Analyt komplexierte Phenoloxidase-Rezeptorkonjugat diffundiert zur räumlich entfernten Elektrodenoberfläche und geht bei Anwesenheit eines Phenoloxidase-Substrats sowie eines Redoxmediators eine signalamplifizierende enzymatisch-elektrochemische Redoxreaktion ein, die ein analytproportionales, voltammetrisches Meßsignal generiert.

Eine zweite erfindungsgemäße Ausführungsform basiert auf einer (quasi)homogenen Affinitätsreaktion. Lokal getrennt von einem immobilisierten modifizierten Liganden erfolgt zunächst eine affine Reaktion des Analyten mit Phenoloxidase-konjugiertem Rezeptor, der im Überschuß vorliegt. Beim Diffundieren zur räumlich entfernten Redoxelektrode passieren die mit dem Phenoloxidase-konjugierten Rezeptor komplexierten Analytmoleküle und der überschüssige Phenoloxidase-konjugierte Rezeptor einen Abschnitt mit immobilisiertem Liganden, an dem der unkomplexierte Anteil gebunden wird, so daß an der Elektrodenoberfläche bei Anwesenheit eines Phenoloxidase-Substrates sowie eines Redoxmediators ausschließlich der analytkomplexierte Phenoloxidase-konjugierte Rezeptoranteil eine signalamplifizierende enzymatisch-elektrochemische Redoxreaktion eingeht.

Eine dritte erfindungsgemäße Ausführungsform geht zunächst von einer affinen Konkurrenzreaktion zwischen Analyt und Phenoloxidase-Ligandkonjugat um die Bindungsstellen eines immobilisierten Rezeptors aus. Der Anteil des Phenoloxidase-Ligandkonjugats, der aufgrund der Analytkomplexierung mit dem Rezeptor nicht gebunden wurde, diffundiert zur räumlich entfernten Redoxelektrode und geht bei Anwesenheit eines Phenoloxidase-Substrats sowie eines Redoxmediators eine signalamplifizierende enzymatisch-elektrochemische Redoxreaktion ein.

Eine vierte, erfindungsgemäß besonders bevorzugte Ausführungsform sieht anstelle der Phenoloxidase in den oben beschriebenen erfindungsgemäßen Ausführungen als Markerenzym ein hydrolysierendes Enzym, vorzugsweise eine Phosphatase oder Galactosidase, vor. Die Phenoloxidase hingegen wird direkt auf der Redoxelektrodenoberfläche in Form einer katalytisch aktiven Schicht immobilisiert, die dann in der Lage ist, die als Markerenzym verwendete Hydrolase über eines ihrer Hydrolyseprodukte, das gleichzeitig ein effizientes Enzymsubstrat der Phenoloxidase ist, bei Anwesenheit eines Redoxmediators signalamplifizierend in die enzymatisch-elektrochemische Detektion einzubringen.

Letztlich wird entweder die als Markerenzym genutzte Phenoloxidase direkt oder als vermittelnde katalytische Schicht bei Anwesenheit eines geeigneten Enzymsubstrats sowie eines Redoxmediators zur eigentlichen Indikationsreaktion an einer kathodisch polarisierten Redoxelektrode genutzt. Dabei setzt die Phenoloxidase bestimmte Enzymsubstrate, vorzugsweise Phenol, m-Kresol, p-Kresol, 2,4-Xylenol, p-Chlorphenol oder Catechol zu einem elektrochemisch aktiven Produkt um, das wiederum voltammetrisch mittels eines reversiblen reduzierten chinoiden Redoxfarbstoffes, Chinons, redoxaktiven Metallkomplexes oder elektrisch leitfähigen redoxaktiven Polymers zu einem Ausgangssubstrat für Phenoloxidase reduziert wird. D.h., das regenerierte Ausgangssubstrat steht zusätzlich für die enzymatische Oxidationsreaktion zur Verfügung. Überraschenderweise führt diese zyklische Substratregenerierung bzw. chemische Signalamplifizierung im Vergleich zu bekannten amperometrischen Detektionssystemen zu einer um ca. drei Größenordnungen niedrigeren Nachweisgrenze.

Der erfindungsgemäße enzymatisch-elektrochemische Einschritt-Affinitätssensor besteht enweder aus einer auf einem planaren Trägerstreifen 1 aufgedruckten voltammetrischen Meßkette 22 oder aus zwei in unmittelbarer Nähe auf einem gemeinsamen Trägerstreifen aufgedruckten identischen voltammetrischen Meßketten 22, 23, die in beiden Ausführungen mit einer Folge von jeweils mit unterschiedlichen für das erfindungsgemäße Affinitätsassay erforderlichen Reagenzien imprägnierten Schichten bedeckt sind. In der zweifachen Ausführungsform dient einer der beiden Teilsensoren auf dem gemeinsamen Trägersubstrat als eigentliches Indikationssystem, während der andere Teilsensor als Funktionstest dient.

Die voltammetrische Meßkette der Sensoren besteht jeweils aus einer Redoxelektrode 3, deren Oberfläche durch einen chinoiden Redoxfarbstoff, ein Chinon, einen redoxaktiven Komplex des Eisens, Rutheniums, Cobalts, Osmiums, Mangans oder Wolframs, ein Metallocen, ein Phthalocyanin oder ein elektrisch leitfähiges Redoxpolymer wie Polyanilin, Polypyrrol, Poly-o-phenylendiamin oder Polyacetylen modifiziert ist und einer sie umgebenden Ag/AgCl - Pseudoreferenzelektrode 4. Die Elektroden einschließlich ihrer Kontaktbahnen 2 für den Anschluß an einen Potentiostaten bzw. ein Handmeßgerät sowie die erforderliche Isolierungsschicht 5 zwischen den Kontaktbahnen werden mittels Siebdruck auf den planaren Trägerstreifen aufgebracht. Die modifizierte Redoxelektrode ist gegen die Pseudoreferenzelektrode katodisch polarisiert.

Unmittelbar über den Elektroden werden in engem Kontakt mehrere aufeinanderfolgende Schichten mittels eines flexiblen Fixierrahmens 6, 7 aus Kunststoff auf die Elektroden gedrückt, die durch die Verwendung entsprechender poröser Materialien bzw. deren Modifizierung zielgerichtet die Diffusion oder Kapillarkräfte begünstigen oder zeitbegrenzt behindern. Als diffusionsbegünstigende bzw. die Kapillarkräfte fördernde Materialen, die auch mit entsprechenden Puffersubstanzen versehen sind, werden vorzugsweise Zellulose, Polysilikate, linear vernetzte Hydrogele oder eine Mischung der genannten Materialien verwendet, die gegebenfalls mit zusätzlichen hydrophilen Komponenten, vorzugsweise mit einem Polysaccharid, Polyalkohol, Polyetheralkohol oder anorganischem Salz versehen werden. Als Diffusionsbarriereschicht, die zeitlich begrenzt als solche wirken soll, dient vorzugsweise ein hydrophobiertes Papier. Um einen effizienten Anreicherungseffekt zu erzielen, sind in einer bevorzugten Ausführungsform die Flächen der Schichten zur Elektrodenoberfläche hin so gestaltet, daß sie sich in ihrer Fläche verringern.

Zum Meßmedium hin werden die Schichten durch eine wasserundurchlässige Folie 9 bzw. Membran, vorzugsweise aus PTFE, Polycarbonat oder einer Kautschukverbindung teilweise bedeckt, die mittels eines ringförmigen porösen Spacers 10 gegen die Abdeckung 8 eines Fixierrahmens distanziert ist. Der Fixierrahmen 6, 7, der einen zylindrischen Hohlraum zur Aufnahme der Schichten enthält und diese gegen ein seitliches Eindringen des Meßmediums schützt, weist in seiner Abdeckung 8 eine kreisförmige Fläche auf. Letztere weist vorzugsweise regelmäßig angeordnete Öffnungen zum Eintritt des Meßmediums auf und ist in ihrem Durchmesser kleiner als der der Folie. Unmittelbar nach der Folie, deren Durchmesser wiederum kleiner als der des zylindrischen Hohlraums des Fixierrahmens ist, ist eine Probeaufnahme-und Reservoirschicht 11 angeordnet, die aus einem schnell wasseraufnehmenden und gut quellbaren Material, vorzugsweise aus einer Zelluloseschicht besteht, die mit einem natürlichen oder synthetischen Hydrogel wie Agar, Gelatine, Pektin, Dextrin, Polyacrylamid oder Polyurethan imprägniert ist. Bei der Aufgabe einer wäßrigen Probe erfolgt durch die Öffnungen der Fixierrahmenabdeckung 8 und den ringförmigen Spacer 10 die Diffusion der Probe in die Probeaufnahme- und Reservoirschicht 11, bis diese aufgrund ihrer Quellung und der damit einhergehenden Volumenvergrößerung die Folie 9 auf die Eintrittsöffnungen der Fixierrahmenabdeckung 8 drückt, so daß die Probenachführung unterbunden wird. Auf diese Weise wird ein definiertes Probevolumen geschaffen.

Der Probeaufnahme- und Reservoirschicht 11 folgt eine Schicht 12, die entweder mit frei diffusionsfähigem Phenoloxidase-Rezeptorkonjugat oder mit Phenoloxidase-konjugiertem modifizierten Liganden versehen ist, und der sich eine diffusionshemmende Schicht 13 anschließt.
Im Fall des diffusionsfähig vorliegenden Phenoloxidase-Rezeptorkonjugats erfolgt eine affine Reaktion mit dem Analyten, während im Fall des diffusionsfähig vorliegenden Phenoloxidase-Ligandkonjugats lediglich eine homogene Verteilung des Analyten in dieser Schicht angestrebt wird. Die Inkubationsdauer wird in beiden Fällen von der Zeitdauer bestimmt, die zum Durchbrechen der nachfolgenden Diffusionsbarriere 13 erforderlich ist.

Die darauffolgende Schicht 14 enthält entsprechend der beiden unterschiedenen Fälle an die Festphase immobilisiert entweder einen Liganden oder einen Rezeptor, der wiederum eine Diffusionsbarrierenschicht 15 folgt. Im ersten Fall wird der Anteil des Phenoloxidase-Rezeptorkonjugats, das in der vorangegangen affinen Reaktion keinen Analyten binden konnte, an dem immobilisierten Liganden gebunden, während das mit Analyt komplexierte Phenoloxidase-Rezeptorkonjugat durch diese Schicht hindurch diffundiert, sobald die Diffsionsbarriere durchbrochen wird. Im zweiten Fall konkurrieren Analyt und das Phenoloxidase-Ligandkonjugat um die Bindungsstellen des immobilisierten Rezeptors. Aufgrund der höheren Affinität des Analyten und der begrenzten Menge an Rezeptor wird analytproportional der überschüssige Anteil des Phenoloxidase-Ligandkonjugats nach dem Durchbrechen der Diffusionsbarriere 15 weiterdiffundieren.

In der sich anschließenden letzten Schicht 16 ist zur Anreicherung entweder ein Rezeptor gegen das mit Analyt komplexierte Phenoloxidase-Rezeptorkonjugat oder ein Rezeptor gegen das Phenoloxidase-Ligandkonjugat an der Festphase immobilisiert. Diese Schicht 16 befindet sich unmittelbar vor der Elektrodenoberfläche und wird umgeben von einer kreisförmigen, durch eine Diffusionsbarriere 17 getrennten, das Phenoloxidase-Substrat enthaltenden Schicht 18. Nach einer Inkubationszeit, die zur Anreicherung entweder des mit Analyt komplexierten Phenoloxidase-Rezeptorkonjugats oder des mit Analyt komplexierten Phenoloxidase-Ligandkonjugats erforderlich ist, dringt das Substrat nach dem Durchbrechen der Diffusionsbarriere 17 in die elektrodennahe Schicht 16 ein, und es wird die eigentliche enzymatisch-elektrochemische Indikationsreaktion via Redoxmediator, der an der Elektrodenoberfläche 3 durch Adsorption, physikalischen Einschluß oder kovalent gebunden wird, an der kathodisch polarisierten Redoxelektrode 3 erfolgen.

Eine zweite erfindungsgemäße Ausführungsform des enzymatisch elektrochemischen Einschritt-Affinitätssensors weist unmittelbar nach der Probeaufnahme- und Reservoirschicht 11 eine Schicht 19 mit Rezeptor oder modifiziertem Liganden auf, der an der Festphase immobilisiert und entweder mit Phenoloxidase-Ligandkonjugat oder Phenoloxidase-Rezeptorkonjugat komplexiert ist. Nach der Diffusion der Probeflüssigkeit aus der Probeaufnahme- und Probereservoirschicht 11 erfolgt eine Verdrängungsreaktion durch den Analyten, so daß entweder der Analyt am immobilisierten Rezeptor anbindet und das Phenoloxidase-Ligandkonjugat verdrängt oder der Analyt mit dem zuvor am immobilisierten Liganden gebundenen Phenoloxidase-Rezeptorkonjugat komplexiert. Der daraus resultierende diffusionsfähige Anteil an entweder Phenoloxidase-Ligandkonjugat oder an analytkomplexiertem Phenoloxidase-Rezeptorkonjugat diffundiert nach Durchbrechen der Diffusionsbarriere 13 wiederum in die elektrodennahe Schicht 16, die wie bereits oben beschrieben aufgebaut ist.
Eine dritte erfindungsgemäße Ausführungsform des enzymatisch elektrochemischen Einschritt-Affinitätssensors sieht anstelle der Phenoloxidase als Markerenzym die Verwendung eines hydrolysierenden Enzyms vor und weist im Unterschied zu den oben beschriebenen Ausführungsformen als elektrodennahe Schicht eine direkt auf der Elektrodenoberfläche immobilisierte Phenoloxidaseschicht 21 zusätzlich auf.
Die mit dem enzymatisch-elektrochemischen Affinitätsassay sowie dem dazugehörigen enzymatisch-elektrochemischen Einweg- und Einschritt-Affinitätssensor erzielten Vorteile bestehen insbesondere darin, daß eine äußerst sensitive, elektrisch quantifizierbare Detektion, insbesondere auch kleiner Analytmoleküle erfolgen kann, wobei das reagenzund separationsfreie Einschritt-Meßsystem eine einfache Handhabung ermöglicht.

Einen weiteren Vorteil bietet die Verwendung zweier identischer und eng benachbarter Teilsensoranordnungen auf dem selben Trägersubstrat. Aufgrund der definierten diffusionsfähigen Analytkonzentration, die die Probeaufnahme-und Reservoirschicht 11 der Funktionstestanordnung im Unterschied zu der eigentlichen Indikationsanordnung enthält, wird der kathodische Meßstrom dieses Teilsensors gegenüber dem Indikationsteilsensor um einen definierten Differenzbetrag höher sein. Damit kann neben der eigentlichen Analytbestimmung gleichzeitig während des Meßvorgangs eine Funktionskontrolle vorgenommen und die Zuverlässigkeit der Messung erhöht werden.

Die Erfindung wird in den nachfolgenden Beispielen 1 - 3 zur Bestimmung des Modellanalyten 2,4 Dinitrophenol (DNP) und im Beispiel 4 zur Bestimmung von IgA sowie anhand der Figuren 1 bis 5b näher erläutert ohne sie darauf zu beschränken; es zeigen:
- Fig.1: eine perspektivisch dargestellte Ausführungsform des erfindungsgemäßen enzymatisch - elektrochemischen Einschritt-Affinitätssensors
- Fig.2: eine Schnittdarstellung entlang der Linie A-A' in Fig.1 durch eine Variante des erfindungsgemäßen enzymatisch - elektrochemischen Einschritt-Affinitätssensors gemäß Anspruch 7
- Fig.3: eine perspektivisch dargestellte Ausführungsform des erfindungsgemäßen enzymatisch - elektrochemischen Einschritt-Affinitätssensors mit einem zweiten identischen Sensorkanal auf dem Support zur Kalibrier- und Funktionskontrolle
- Fig.4: eine Schnittdarstellung entlang der Linie A-A' in Fig.1 durch eine Variante des erfindungsgemäßen enzymatisch - elektrochemischen Einschritt-Affinitätssensors gemäß Anspruch 8
- Fig.5a: eine Schnittdarstellung entlang der Linie A-A' in Fig.1 durch eine Variante des erfindungsgemäßen enzymatisch - elektrochemischen Einschritt-Affinitätssensors gemäß Anspruch 9
- Fig.5b: eine Schnittdarstellung entlang der Linie A-A' in Fig.1 durch eine Variante des erfindungsgemäßen enzymatisch - elektrochemischen Einschritt-Affinitätssensors gemäß Anspruch 10

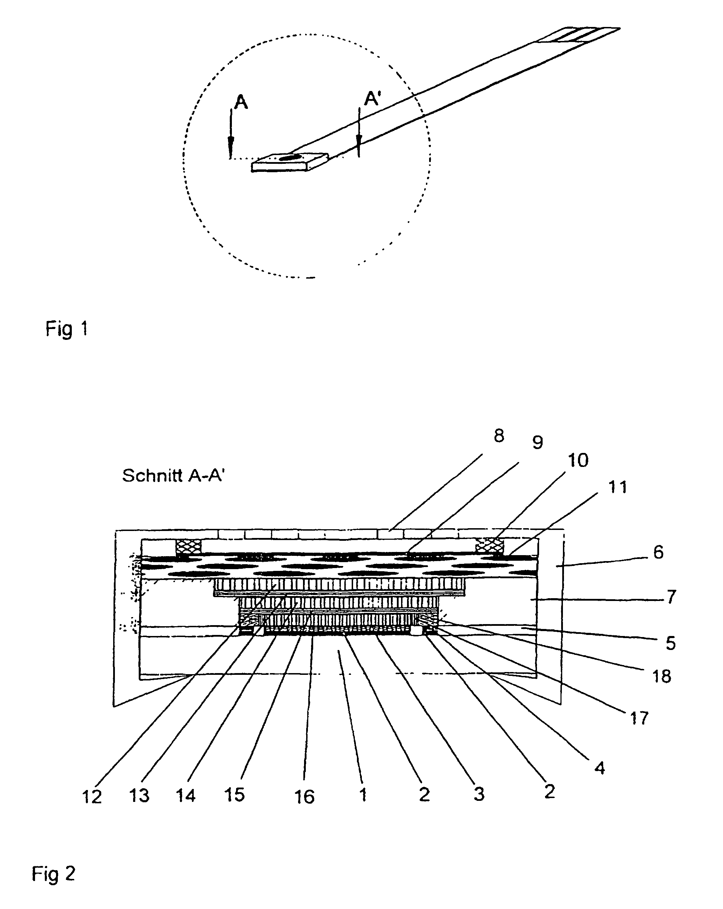

### Beispiel 1 (Fig. 1,2)

Mittels polymerer Dickschichtpasten werden auf ein Glasfaser-Epoxidharz-Support 1 nacheinander Kohlenstoffkontaktbahnen 2, eine N-Methylphenazinium-Reineckat-modifizierte Kohlenstoffarbeitselektrode 3, eine Silber/Silberchlorid (Ag/AgCl)-Referenzelektrode 4, die in Form eines quadratischen Bandes die Arbeitselektrode 3 umgibt, und eine Isolierschicht 5, die außer den Flächen der Arbeits- und Referenzelektrode sowie Steckkontaktflächen den Support bedeckt, gedruckt und bei 90°C gehärtet. Unmittelbar auf der Meßfensterfläche, in der die Arbeitselektrode 3 und Referenzelektrode 4 angeordnet sind, wird ein Fixierrahmen aus Kunststoff 6, 7 durch entsprechende Ausformungen auf dem Support 1 aufgeklemmt. Der Fixierrahmen enthält einen zylinderförmigen Hohlraum und weist in seiner oberen Abdekkung 8 auf einer begrenzten Kreisfläche von 1,5 mm im Durchmesser regelmäßig angeordnete Öffnungen mit Durchmessern von 0.1 mm auf. Der Fixierrahmen wird mit einer Folge unterschiedlicher Schichten dicht bepackt: Unmittelbar unter der perforierten Kreisfläche der Fixierrahmenabdeckung 8 befindet sich eine Siloprenfolie 9 (ø 3 mm), die mit einem ringförmigen Spacer 10 (ø_{A} 3 mm, ø_{I} 2mm) aus Filterpapier gegen die Abdeckung auf Distanz gehalten wird. Die Siloprenfolie wurde durch entsprechende Katalysatorzugabe (Vernetzer KA-1, Bayer AG) zum Silopren K1000 (Fluka) auf einer PTFE-Unterlage zu einer Folie polykondensiert und als Blättchen ausgestanzt. Der Siloprenfolie folgt eine quellbare Schicht 11 mit einem Durchmesser von 5 mm, die aus einem mit ®SANWET IM 3900 G (Hoechst AG) beschichteten Filterpapier besteht und als Probeaufnahme- und Reservoirschicht dient.
Diese wie auch die nachfolgenden Filterpapierschichten wurden aus Blauband-Rundfilterpapier (Schleicher & Schüll 589/3), die zuvor mit einer wäßrigen Lösung aus 2 Vol % Dextran, MG 70.000 (Sigma), 10 Vol % Glycerol und 0.1 M Phosphatpuffer, pH 6,8 imprägniert wurden, hergestellt. Das Rundfilterpapier hatte einen Durchmesser von 55 mm und wurde nach den jeweiligen Modifizierungsprozeduren passend zu dem jeweiligen Durchmesser des zylindrischen Hohlraums des Fixierrahmens (5mm - 1 mm) ausgestanzt.
An die Probeaufnahme- und Reservoirschicht schließt sich eine Filterpapierschicht 12 (ø 3 mm) an, die frei diffusionsfähiges DNP-L-Lysin - Tyrosinasekonjugat enthält. ε-2,4 DNP -L-Lysin (Sigma) wurde analog der gemischten Anhydridmethode nach Jung et.al.,J. Agric. Food Chem., 37 (1989), 1183 an Tyrosinase, 2000 U/mg (Sigma) gekoppelt. Unmittelbar darunter ist eine mit Silikon leicht hydrophobierte Papierschicht 13 (ø 3 mm) als Diffusionsbarriere angeordnet, der eine Filterpapierschicht 14 (ø 1 mm) mit gerichtet immobilisierten Fängerantikörpern folgt. Zur Herstellung letztgenannter Schicht wurde das Filterpapier zunächst einer Hydrolysierungsprozedur unterzogen. Die resultierenden OH-Gruppen wurden mit Carbonyldiimidazol aktiviert und mittels Bernsteinsäuredihydrazid an die zuvor mit Perjodat oxidierten Kohlenhydrate des Fc-Teils der Antikörper (2 ml) kovalent gebunden. Zur Präparierung wurden die bekannten Prozeduren, wie in M.B. Wilson und P.K. Nakane: "Immunofluorescence and Releted Staining Techniques", Elsevier, North Holland Biomedical Press, S. 215-224, ....und in G.T. Hermanson, A.K. Mallia und P.K. Smith: Immobilized Affinity Ligand Techniques, Academic press, San Diego, CA, 1992 beschrieben, genutzt. Als Antikörper dienten kommerziell verfügbare polyklonale anti-DNP Antikörper aus Kaninchen (Sigma). Zur Minimierung der unspezifischen Bindung wurde die Immobilisierungsmatrix mit einer 1%igen Casein-Phosphatpuffer/KCl-Lösung abgeblockt.
Daran schließt sich wieder eine hydrophobierte Papierschicht 15 (ø 1 mm) und nochmals die gleiche mit Antikörpern immobiliserte Filterpapierschicht 16 (ø 1 mm) an, die dann unmittelbar auf der Arbeitselektrodenoberfläche aufliegt. Diese Filterpapierschicht 16 bildet gleichzeitig den elektrodennahen Raum und wird von einem mit 0,1 mM Catechol imprägnierten Filterpapierring 18 (ø_{A} 2 mm, ø_{I} 1 mm) umgeben, dessen Innendurchmesser mit Silikon imprägniert ist und damit eine Diffusionsbarriere 17 schafft.

Bei Aufgeben eines wäßrigen Probetropfens auf die perforierte Fixierrahmenabdeckung 8 diffundiert die Probeflüssigkeit via Spacer 9 zunächst in die Probeaufnahme- und Reservoirschicht 11. Die dadurch verursachte Quellung der Probeaufnahme- und Reservoirschicht 11 führt dazu, daß die Siloprenfolie 9 an die perforierte Fixierrahmenabdeckung 8 gedrückt wird und diese verschließt. Die wäßrige Probe diffundiert durch die darauffolgende Dinitrophenol-Tyrosinasekonjugat enthaltende Filterpapierschicht 12 und mobilisiert das Konjugat. Nach dem Durchbrechen der als Diffusionsbarriere dienenden hydrophobierten Papierschicht 13 gelangt sowohl das Konjugat als auch der Analyt in eine weitere Filterpapierschicht 14, die den darin immobilisierten Fängerantikörper enthält. In der heterogenen Immunoreaktion konkurrieren nun das mit Tyrosinase konjugierte Hapten und das Dinitrophenol um die Bindungsstellen der gerichtet immobilisierten Antikörper. Aufgrund der höheren Affinität der Analytmoleküle gegenüber dem Konjugat wird die Komplexierung des Analyten bevorzugt. Je mehr Analyt sich in der Probe befindet, um so weniger DNP-L-Lysin - Tyrosinasekonjugat wird abgebunden. Durch die nachfolgend angeordnete hydrophobe Papierschicht 15 wird eine ausreichende Inkubationszeit für die kompetitive Immunoreaktion gesichert. Nach dem Überwinden dieser Diffusionsbarriere gelangt das nicht gebundene Hapten-Enzymkonjugat in die elektrodennahe Schicht 16, die wiederum mit immobilisierten Antikörpern gegen DNP versehen ist, und wird über die Bindung an die Antikörper direkt vor der Elektrodenoberfläche 3 angereichert. Aufgrund der wäßrigen Probe wird das als Enzymsubstrat verwendete Catechol aus dem Filterpapierring 18, der die elektrodennahe Schicht umschließt, herausgelöst und initiiert seine enzymkatalysierte Umsetzung. Die Oxidation des Catechols erfolgt zu o-Chinon, das durch das kathodisch reduzierte N-Methylphenazinium auf der Elektrodenoberfläche 3 wieder zu Catechol reduziert wird. Der aus dieser zyklischen enzymatisch-elektrochemischen Substratregenerierung resultierende kathodische Meßstrom, der mittels Square-Wave-Voltammetrie bei -160 mV vs. die interne Ag/AgCl-Pseudoreferenzelektrode 4 als Peakstrom detektiert wird, ist der DNP-Konzentration proportional. Der Meßbereich dieses Einschritt-Immunosensors liegt für DNP zwischen 0.5 und 20 µg/l.

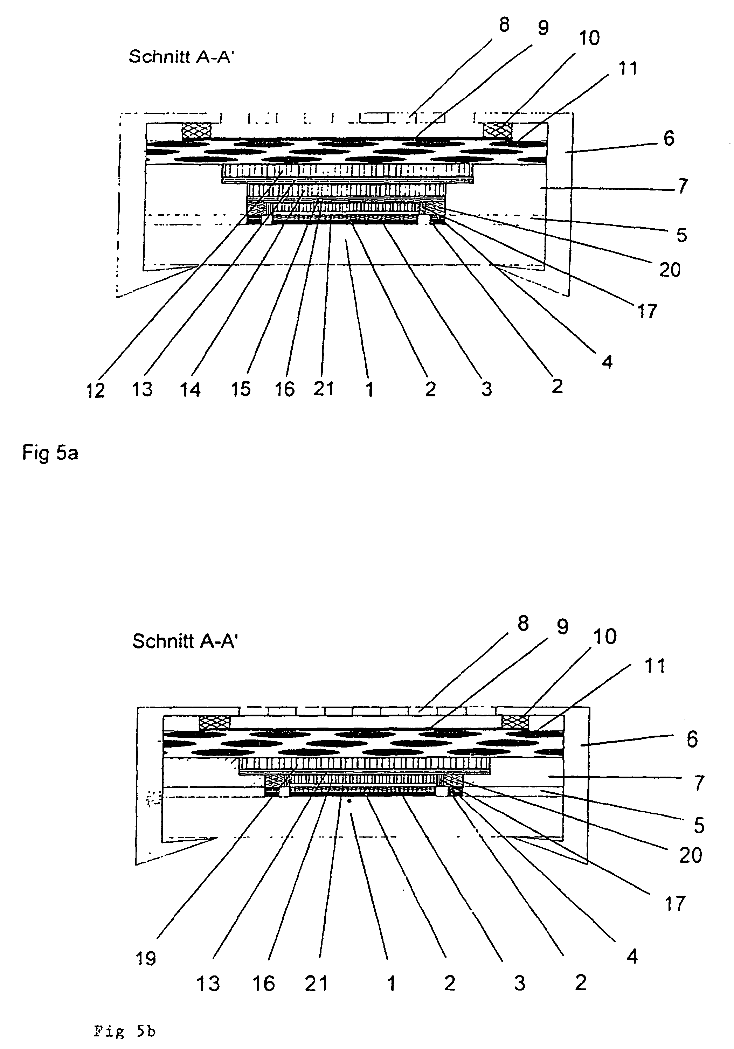

### Beispiel 2 (Fig. 5a)

Das zweite Ausführungsbeispiel basiert auf dem gleichen Sensoraufbau wie in Beispiel 1 beschrieben, verwendet jedoch alkalische Phosphatase 150 U/mg (Sigma) zum Markieren des Haptens (DNP-L-Lysin). Dementsprechend wird der Filterpapierring 20 mit 1mM Phenylphosphat imprägniert und zwischen der elektrodennahen Schicht 16 und der Arbeitselektrodenoberfläche 3 eine zusätzliche Schicht 21, die Tyrosinase, 2000 U/mg (Sigma) in einer PUR-Hydrogelschicht immobilisiert (Kotte et.al., Anal. Chem., 67 (1995), 65) enthält, eingebracht. Die immunochemische Reaktion ist analog Beispiel 1, wobei die eigentliche Indikationsreaktion über die Zwei-Enzymsequenz alkalische Phosphatase-Tyrosinase erfolgt: Das nicht gebundene Phosphatase-DNP-L-Lysin Konjugat, das in die elektrodennahe Schicht 16 gelangt und durch die darin immobilisierten anti-DNP-Antikörper gebunden und damit angereichert wird, hydrolysiert das aus dem umgebenden Filterpapierring ausdiffundierende Phenylphosphat zu Phenol. Das Phenol wird schließlich durch die nachfolgende Tyrosinaseschicht zu o-Chinon oxidiert, das durch das kathodisch reduzierte N-Methylphenazinium auf der Elektrodenoberfläche 3 wieder zu Catechol reduziert wird. Der aus dieser zyklischen enzymatisch-elektrochemischen Substratregenerierung resultierende kathodische Meßstrom, der mittels Square-Wave-Voltammetrie bei -160 mV vs. die interne Ag/AgCl-Pseudoreferenzelektrode 4 als Peakstrom detektiert wird, ist der DNP-Konzentration proportional. Der Meßbereich dieses Einschritt-Immunosensors liegt für DNP zwischen 0.1 und 10 µg/l.

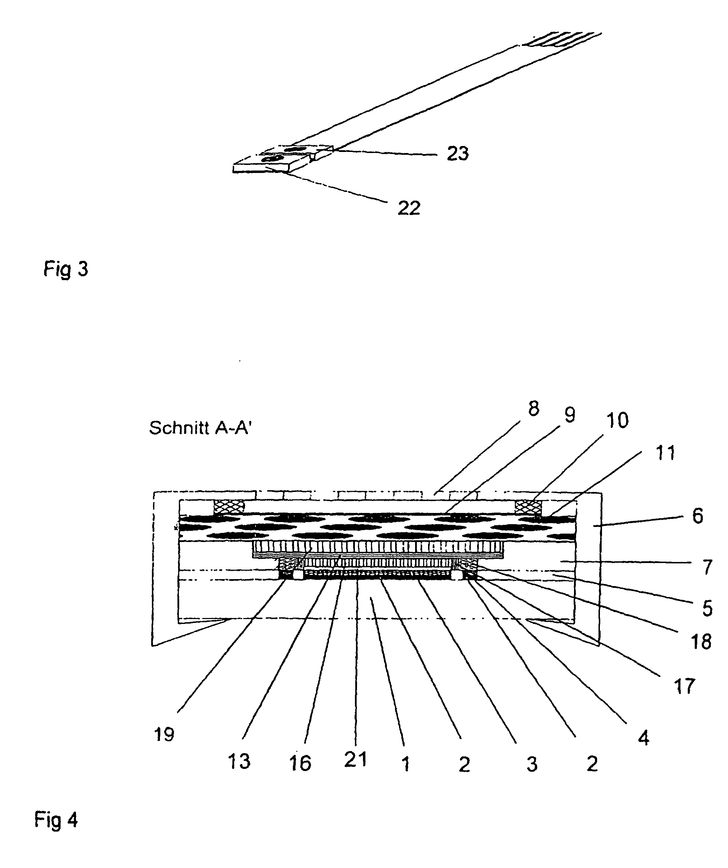

### Beispiel 3 (Fig. 3)

Dieses Beispiel beschreibt die Verwendung zweier auf einem Support nacheinander angeordneter Immunosensorsysteme 22, 23, die wie im Beispiel 1 beschrieben aufgebaut sind. Von den unabhängig voneinander funktionierenden "Teilsensoren" bildet einer das eigentliche Indikationssystem 22, während der andere als Referenz-und Funktionstestsystem 23 dient. Das Referenz- und Funktionstestsystem unterscheidet sich vom Indikationssystem dadurch, daß seine Probe- und Reservoirschicht mit einer definierten Analytkonzentration von 1 µg/l imprägniert ist, so daß man nach Aufgabe eines entsprechenden Probetropfens bzw. durch Eintauchen des Sensors in die Probe am Referenz- und Kontrolltestsystem 22 aufgrund des vorrätigen Analyten einen definierten "Mindestsignalstrom" erwarten kann zu dem sich der eigentliche Meßstrom addiert. In Abwesenheit von Analyt in der Probe beträgt dieser 5...8 nA. Der Meßstrom des Indikationssystems 22, der analytabhängig zwischen 0.1 und 20 nA liegt, fällt um diesen Mindestsignalstrombetrag geringer aus. Auf diese Weise kann zum einen die Sensorfunktion während des Meßvorganges selbst kontrolliert und zum anderen durch die Verrechnung beider Meßströme bei der Signalauswertung die Zuverlässigkeit der Messung erhöht werden.

### Beispiel 4 (Fig. 2)

Dieses Beispiel beschreibt einen Affinitätssensor auf der Grundlage des Lektins Jacalin, einem Glycoprotein (Kumar, G.S., et.al., Biosci.,4(1982), 257-61), als selektive affine Komponente zur Bestimmung von IgA im Blutplasma (Kondoh, H., et al., Immunol. Meth., 88 (1986), 171-73). Der prinzipielle Sensoraufbau basiert auf dem Aufbau, der in Beispiel 1 beschrieben ist. Allerdings wurde in Schicht 12 frei diffusionsfähiges IgA-Tyrosinase Konjugat verwendet. Die Tyrosinase (Sigma) wurde analog der in Beispiel 1 verwendeten Methode an menschliches (Plasma) IgA (CALBIOCHEM) gekoppelt. Außerdem wurde in den Schichten 14 und 16 anstelle des Fängerantikörpers Jacalin (Pierce) immobilisiert. Dazu wurde Blauband-Rundfilterpapier (Schleicher & Schüll 589/3, Durchmesser: 55 mm), mittels Carbonyldiimidazol aktiviert (Hissey, P.H., et al., Immunol. Meth. 78 (1985), 211-16), so daß das Jacalin via NH₂ -Gruppen kovalent gebunden wurde. Für eine Filterpapierfläche von ca. 23 cm² wurden 3 mg Jacalin in einem Milliliter 0.1 M Boratpuffer (pH 8,5) verwendet. Zur kovalenten Bindung wurde das CDA-aktivierte Filterpapier über 24 Stunden bei 8°C mit der Jacalinlösung inkubiert und danach mit 0.1 M Phosphatpufferlösung (pH 6,8), die 2 Vol % Dextran MG 70.000 (Sigma), 10 Vol % Glycerol (Sigma) enthielt, gespült und getrocknet. Danach wurde das Papier mit einer 1% igen Casein-Phosphatpuffer/KCl-Lösung (0,1 M) behandelt, getrocknet und mit einem Durchmesser von 1 mm ausgestanzt und wie in Beispiel 1 beschrieben als Schichten 14 und 16 verwendet.
Bei Aufgeben eines Blutplasmatropfens auf die perforierte Fixierrahmenabdeckung 8 diffundiert die Probeflüssigkeit via Spacer 9 zunächst in die Probeaufnahme- und Reservoirschicht 11. Die dadurch verursachte Quellung der Probeaufnahme- und Reservoirschicht 11 führt dazu, daß die Siloprenfolie 9 an die perforierte Fixierrahmenabdeckung 8 gedrückt wird und diese verschließt. Die wäßrige Probe diffundiert durch die darauffolgende IgA-Tyrosinasekonjugat enthaltende Filterpapierschicht 12 und mobilisiert das Konjugat.
Nach dem Durchbrechen der als Diffusionsbarriere dienenden hydrophobierten Papierschicht 13 gelangt sowohl das Konjugat als auch der Analyt in eine weitere Filterpapierschicht 14, die das darin immobilisierte Jacalin enthält, In der heterogenen Immunoreaktion konkurrieren nun das mit Tyrosinase markierte IgA und das IgA der Probe um die Bindungsstellen des immobilisierten Lektins. Aufgrund der Gleichgewichtsreaktion erfolgt die Komplexierung analytproportional. Je mehr Analyt sich in der Probe befindet, um so weniger IgA-Tyrosinasekonjugat wird abgebunden. Durch die nachfolgend angeordnete hydrophobe Papierschicht 15 wird eine ausreichende Inkubationszeit für die kompetitive Immunoreaktion gesichert.
Nach dem Überwinden dieser Diffusionsbarriere gelangt das nicht gebundene IgA-Enzymkonjugat in die elektrodennahe Schicht (16), die identisch zu Schicht 14 immobilisiertes Lektin aufweist, so daß das in Schicht 14 nicht abgebundene IgA-Tyrosinasekonjugat nun durch das immobilisierte Jacalin gebunden und damit vor der Elektrodenoberfläche 3 angereichert wird. Aufgrund der wäßrigen Probe wird das als Enzymsubstrat verwendete Catechol aus dem Filterpapierring 18, der die elektrodennahe Schicht umschließt, herausgelöst und initiiert seine enzymkatalysierte Umsetzung. Die Oxidation des Catechols erfolgt zu o-Chinon, das durch das kathodisch reduzierte N-Methylphenazinium auf der Elektrodenoberfläche 3 wieder zu Catechol reduziert wird. Der aus dieser zyklischen enzymatisch-elektrochemischen Substratregenerierung resultierende kathodische Meßstrom, der mittels Square-Wave-Voltammetrie bei -160 mV vs. die interne Ag/AgCl-Pseudoreferenzelektrode 4 als Peakstrom detektiert wird, ist der IgA-Konzentration proportional.

### Bezugszeichenliste

- 1: Träger
- 2: Kontaktbahnen der Elektroden
- 3.: mediatormodifizierte Redoxelektrode
- 4: Pseudoreferenzelektrode
- 5: Isolierschicht
- 6: Fixierrahmen
- 7: Fixierrahmen
- 8: obere Abdeckung des Fixierrahmens mit perforierter Fläche
- 9: Folie
- 10: Spacer
- 11: Probeaufnahme- und Reservoirschicht
- 12: Schicht, die frei diffusionsfähigen Phenoloxidase- oder Hydrolasemarkierten Liganden oder Rezeptor enthält
- 13: Diffusionsbarriereschicht
- 14: Schicht, enthaltend einen immobilisierten Liganden oder Rezeptor
- 15: Diffusionsbarriereschicht
- 16: elektrodennahe Schicht, enthaltend einen immobilisierten Liganden oder Rezeptor
- 17: Diffusionsbarriereschicht
- 18: Enzymsubtratschicht, enthaltend ein Substrat für die Phenoloxidase
- 19: Schicht, die Phenoloxidase- oder Hydrolasemarkierte immobilisierte Rezeptor/Ligand-Komplexe enthält
- 20: Enzymsubstratschicht, enthaltend ein Substrat für die Hydrolase
- 21: elektrodennahe Schicht, enthaltend immobilisierte Phenoloxidase
- 22: Meßsystem (identisch zu 23)
- 23: Meßsystem (identisch zu 22)

## Patentansprüche

1. Enzymatisch-elektrochemischer Einschritt-Affinitätssensor zur quantitativen Bestimmung von Analyten in wäßrigen Medien bestehend aus einem Träger (1), auf dem ein Meßsystem (22) oder zwei nebeneinanderliegende, identisch aufgebaute Meßsysteme (22 und 23) und deren Kontaktbahnen (2) aufgebracht sind, wobei jedes Meßsystem jeweils aus mehreren, aufeinanderfolgenden, über einer mit einem Elektronenmediator modifzierten Redoxelektrode (3) und ihrer Pseudoreferenzelektrode (4) angeordneten Schichten besteht, die mittels eines Fixierrahmens (6) seitlich flüssigkeitsdicht verkapselt sind und dessen obere Abdeckung (8) eine Fläche aufweist, die Öffnungen zur Aufnahme der zu messenden Probe besitzt, wobei die Schichten entweder
a) für den Fall, daß das Markerenzym der affinen Bindungspartner Phenoloxidase ist, eine Schicht (18) mit einem Phenoloxidase-Substrat umfassen oder
b) für den Fall, daß das Markerenzym der affinen Bindungspartner eine Hydrolase ist, eine Schicht (20) mit einem Hydrolasesubstrat und eine zusätzliche elektrodennahe Schicht (21) mit immobilisierter Phenoloxidase umfassen,
neben einer Probeaufnahme- und Reservoirschicht (11), einer elektrodennahen Schicht (16), den Schichten (12) und (14), die affine Bindungspartner enthalten oder einer Schicht (19), die entsprechende immobilisierte Affinitätskomplexe beinhaltet.

2. Sensor nach Anspruch 1,
**dadurch gekennzeichnet,**
**daß** im Fall zweier Meßsysteme (22, 23) das eine Meßsystem das eigentliche Indikationssystem darstellt und das andere Meßsystem zur Kalibrier- und Funktionskontrolle dient.

3. Sensor nach Anspruch 1 oder 2,
**dadurch gekennzeichnet,**
**daß** die Redoxelektrode, die vorzugsweise eine mediatormodifizierte Kohlenstoffelektrode ist, gegen die Pseudoreferenzelektrode, die vorzugsweise eine Ag/AgCl-Elektrode ist, eine Polarisationsspannung zwischen - 300 mV und 100 mV aufweist.

4. Sensor nach einem der Ansprüche 1 bis 3,
**dadurch gekennzeichnet,**
**daß** der Elektronenmediator der modifizierten Redoxelektrode durch Adsorption, physikalischen Einschluß, kovalente Bindung oder in Form eines Redoxpolymers auf der Elektrodenoberfläche fixiert ist.

5. Sensor nach einem der Ansprüche 1 bis 4,
**dadurch gekennzeichnet,**
**daß** der Elektronenmediator ein chinoider Redoxfarbstoff, ein Chinon, redoxaktive Komplexverbindungen des Eisens, Rutheniums oder Wolframs, ein Metallocen, Phthalocyanin oder ein elektrisch leitfähiges Redoxpolymer wie Polyanilin, Polypyrrol, Poly-o-phenylendiamin oder Polyacetylen ist.

6. Sensor nach einem der Ansprüche 1 bis 5,
**dadurch gekennzeichnet,**
**daß** unter der oberen Abdeckung (8) des Fixierrahmens (6) eine, die Perforationen der Abdeckung überlappende, wasserundurchlässige Folie (9) angeordnet ist, die mit einem Spacer (10) auf Distanz zur Abdeckung (8) gehalten wird, und sich nach der Folie (9) die Probeaufnahme- und Reservoirschicht (11) anschließt.

7. Sensor nach einem der Ansprüche 1 bis 6,
**dadurch gekennzeichnet,**
**daß** nach der Probeaufnahme- und Reservoirschicht (11) eine Schicht (12) angeordnet ist, die entweder einen frei diffusionsfähigen, Phenoloxidase-markierten Liganden oder einen frei diffusionsfähigen Phenoloxidase-markierten Rezeptor enthält, anschließend eine Diffusionsbarriereschicht (13) folgt, danach eine Schicht (14) mit einem immobilisierten Liganden oder einem immobilisierten Rezeptor angeordnet ist, anschließend eine zweite Diffusionsbarriereschicht (15) folgt und danach sich als letzte Schicht die elektodennahe Reaktionsschicht (16) anschließt, an der entweder Ligand oder Rezeptor immobilisiert sind, und die von einer durch eine Diffusionsbarriere (17) getrennten Enzymsubstratschicht (18), die ein Substrat für die Phenoloxidase enthält, umschlossen wird.

8. Sensor nach einem der Ansprüche 1 bis 6,
**dadurch gekennzeichnet,**
**daß** nach der Probeaufnahme- und Reservoirschicht (11) eine Schicht (19) angeordnet ist, die entweder einen immobilisierten Rezeptor, der mit einem Phenoloxidase-markierten Liganden komplexiert ist, oder einen immobilisierten Liganden, der mit einem Phenoloxidase-markierten Rezeptor komplexiert ist, enthält, danach eine Diffusionsbarriereschicht (13) folgt, und anschließend sich als letzte Schicht die elektrodennahe Reaktionsschicht (16) anschließt, an der entweder Ligand oder Rezeptor immobilisiert sind, und die von einer durch eine Diffusionsbarriere (17) getrennten Enzymsubstratschicht (18), die ein Substrat für die Phenoloxidase enthält, umschlossen wird.

9. Sensor nach einem der Ansprüche 1 bis 6,
**dadurch gekennzeichnet,**
**daß** nach der Probeaufnahme- und Reservoirschicht (11) eine Schicht (12) angeordnet ist, die entweder einen frei diffusionsfähigen, Hydrolase-markierten Liganden oder einen frei diffusionsfähigen Hydrolase-markierten Rezeptor enthält, anschließend eine Diffusionsbarriereschicht (13) folgt, danach eine Schicht (14) mit einem immobilisierten Liganden oder einem immobilisierten Rezeptor angeordnet ist, anschließend eine zweite Diffusionsbarriereschicht (15) folgt, danach sich die elektrodennahe Reaktionschicht (16) anschließt, an der entweder Ligand oder Rezeptor immobilisiert ist, und die von einer durch eine Diffusionsbarriere (17) getrennten Enzymsubstratschicht (20), die ein Substrat für die Hydrolase enthält, umschlossen wird und zwischen Schicht (16) und Redoxelektrode (3) eine zusätzliche elektrodennahe Schicht (21) angeordnet ist, die immobilisierte Phenoloxidase enthält.

10. Sensor nach einem der Ansprüche 1 bis 6,
**dadurch gekennzeichnet,**
**daß** nach der Probeaufnahme- und Reservoirschicht (11) eine Schicht (19) angeordnet ist, die entweder einen immobilisierten Rezeptor, der mit einem Hydrolase-markierten Liganden komplexiert ist, oder einem immobilisierten Liganden, der mit einem Hydrolase-markierten Rezeptor komplexiert ist, enthält, danach eine Diffusionsbarriereschicht (13) folgt, danach sich die elektrodennahe Reaktionsschicht (16) anschließt, an der entweder Ligand oder Rezeptor immobilisiert ist, und die von einer durch eine Diffusionsbarriere (17) getrennten Enzymsubstratschicht (20), die ein Substrat für die Hydrolase enthält, umschlossen wird, und zwischen Schicht (16) und Redoxelektrode (3) eine zusätzliche, elektrodennahe Schicht (21) angeordnet ist, die immobilisierte Phenoloxidase enthält.

11. Sensor nach einem der Ansprüche 1 bis 10,
**dadurch gekennzeichnet,**
**daß** die wasserundurchlässige Folie (9) eine Membran, vorzugsweise aus PTFE, Polyethylen, Polycarbonat oder Kautschukverbindungen ist.

12. Sensor nach einem der Ansprüche 1 bis 11,
**dadurch gekennzeichnet,**
**daß** die Probeaufnahme- und Reservoirschicht (11) quellbar ist und ein natürliches oder synthetisches Hydrogel enthält, vorzugsweise aus Agar, Gelatine, Pektin, Dextrin, Polyacrylat oder Polyurethan.

13. Sensor nach einem der Ansprüche 1 bis 12,
**dadurch gekennzeichnet,**
**daß** als Diffusionsbarriereschicht (13, 15, oder 17) ein hydrophobiertes Papier dient.

14. Sensor nach einem der Ansprüche 1 bis 13,
**dadurch gekennzeichnet,**
**daß** die die affinen Bindungspartner enthaltenden Schichten (12, 14 oder 16), die enzymsubstratenthaltenden Schichten (18 oder 20), die Phenoloxidaseschicht (21) oder die die enzymmarkierten immobilisierten Affinitätskomplexe enthaltende Schicht (19) aus einem, eine hydrophile Komponente enthaltenden, saugfähigen Material besteht, vorzugsweise einer Zellulose, einem Polysilikat, einem linear vernetzten Hydrogel oder einer Mischung der genannten Materialien.

15. Sensor nach Anspruch 14,
**dadurch gekennzeichnet,**
**daß** als hydrophile Komponente ein Polysaccharid, Polyalkohol, Polyetheralkohol, ein anorganisches Salz oder eine Mischung der genannten Komponenten dient.

16. Sensor nach einem der Ansprüche 1 bis 15,
**dadurch gekennzeichnet,**
**daß** als Phenoloxidase Tyrosinase eingesetzt wird.

17. Sensor nach einem der Ansprüche 1 bis 16,
**dadurch gekennzeichnet,**
**daß** als Enzymsubstrat der Tyrosinase Phenol, m-Kresol, p-Kresol, 2,4-Xylenol, p-Chlorphenol oder Catechol eingesetzt wird.

18. Sensor nach einem der Ansprüche 1 bis 6 oder 9 bis 16,
**dadurch gekennzeichnet,**
**daß** als hydrolisierendes Enzym eine alkalische Phosphatase, saure Phosphatase, oder β-Galactosidase eingesetzt wird.

19. Enzymatisch-elektrochemisches Einschritt-Affinitätsassay zur quantitativen Bestimmung von Analyten in wäßrigen Medien,
**dadurch gekennzeichnet,**
**daß** auf die perforierte Fläche der Fixierrahmenabdeckung (8) eines Sensors gemäß den Ansprüchen 1 bis 18, der an einen Potentiostaten oder ein potentiostatisch aufgebautes Handmeßgerät angeschlossen ist, die Probeflüssigkeit aufgetragen wird und zunächst via Spacer (10) in die Probeaufnahme-und Reservoirschicht (11) diffundiert, diese Schicht quillt und dadurch die Folie (9) an die Fixierrahmenabdeckung (8) gedrückt wird, diese verschließt und danach der zu bestimmende Analyt in der Probeflüssigkeit beim Durchlaufen der Schichten des Sensors gemäß den Ansprüchen 1 bis 18 eine affine Bindungsreaktion eingeht, indem,
a) ein Phenoloxidase-markierter Rezeptor, ein Phenoloxidase-markierter Ligand oder der entsprechende Phenoloxidase-markierte Affinitätskomplex mit dem Analyten zur mediatormodifizierten Elektrodenoberfläche (3) diffundiert und die Phenoloxidase ein geeignetes Phenoloxidase-Substrat in Schicht (18) in unmittelbarer Elektrodenoberflächennähe zu einem elektroaktiven Produkt oxidiert, das via reversibel reduziertem Elektronenmediator kathodisch zu einem Ausgangssubstrat der Phenoloxidase reduziert wird oder
b) Phenoloxidase auf der Redoxelektrode (3) als Schicht (21) fixiert wird und ein Edukt, das aus der Reaktion eines in gleicher Weise wie in a) als Marker verwendeten hydrolisierenden Enzyms mit einem geeigneten Hydrolasesubstrat in Schicht (20) resultiert, durch die als katalytische Schicht (21) genutzte Phenoloxidase in unmittelbarer Elektrodenoberflächennähe zu einem elektroaktiven Produkt oxidiert wird, das via reversibel reduziertem Elektronenmediator kathodisch zu einem Ausgangsubstrat der Phenoloxidase reduziert wird, wobei die in beiden Fällen a) oder b) generierte zyklische Substratregenerierung zu einem chemisch amplifizierten, analytproportionalen kathodischen Strom führt, der mit an sich üblichen voltammetrischen Methoden quantifiziert wird.

20. Assay nach Anspruch 19,
**dadurch gekennzeichnet,**
**daß** der zu bestimmende Analyt den Phenoloxidase-markierten Liganden oder Rezeptor in Schicht (12) mobilisiert, eine pseudohomogene Bindungsreaktion zwischen dem Analyten und dem diffusionsfähigen Phenoloxidase-markierten Rezeptor oder Phenoloxidase-markierten Liganden erfolgt, der nichtgebundene Anteil des Rezeptor- oder Ligandkonjugats nach Überwindung der Diffusionsbarriereschicht (13) in Schicht (14) mit immobilisiertem Liganden oder immobilisiertem Rezeptor gebunden wird, und der Analyt-Rezeptorkonjugat-Komplex oder Analyt-Ligandkonjugat-Komplex nach Überwindung der Diffusionsbarriereschicht (15) in die elektrodennahe Reaktionsschicht (16) gelangt, an einen immobilisierten Rezeptor oder Liganden gebunden wird, und die Marker-Phenoloxidase nach der Mobilisierung des Phenoloxidase-Substrats in der Substratschicht (18) und seines Durchdringens der Diffusionsbarriereschicht (17) in die elektrodennahe Reaktionsschicht (16) hinein eine Amplifizierungsreaktion zwischen Phenoloxidase-Substrat und modifizierter Elektrodenoberfläche (3) katalysiert, die ein analytproportionales voltammetrisches Stromsignal liefert.

21. Assay nach Anspruch 19,
**dadurch gekennzeichnet,**
**daß** der zu bestimmende Analyt in der Probeflüssigkeit den Phenoloxidase-markierten Liganden oder Rezeptor in Schicht (12) mobilisiert, Analyt und Phenoloxidase-markierter Ligand oder Phenoloxidase-markierter Rezeptor nach Überwindung der Diffusionsbarriereschicht (13) in Schicht (14) mit immobilisiertem Liganden oder Rezeptor um die verfügbaren Bindungsstellen konkurrieren, und der nichtgebundene Phenoloxidase-markierte Ligand oder Phenoloxidase-markierte Rezeptor nach Überwindung der weiteren Diffusionsbarriereschicht (15) in die elektrodennahe Reaktionsschicht (16) gelangt, an den immobilisierten Rezeptor oder Liganden in Schicht (16) gebunden wird, und die Marker-Phenoloxidase nach der Mobilisierung des Phenoloxidase-Substrats in der Substratschicht (18) und seines Durchdringens der Diffusionsbarriereschicht (17) in die elektrodennahe Reaktionsschicht (16) hinein eine Amplifizierungsreaktion zwischen Phenoloxidase-Substrat und modifizierter Elektrodenoberfläche (3) katalysiert, die ein analytproportionales voltammetrisches Stromsignal liefert.

22. Assay nach Anspruch 19,
**dadurch gekennzeichnet,**
**daß** der zu bestimmende Analyt in der Probeflüssigkeit die Schicht (19) erreicht, die immobilisierten Liganden oder Rezeptor enthält, deren Bindungsstellen mit Phenoloxidase-Rezeptorkonjugat oder Phenoloxidase-Ligandkonjugat, respektive, abgesättigt sind, der Analyt einen Teil des Phenoloxidase-Ligandkonjugats oder des Phenoloxidase-Rezeptorkonjugats verdrängt, und das mit dem Analyten komplexierte Phenoloxidase-Ligandkonjugat oder das mit dem Analyten komplexierte Phenoloxidase-Rezeptorkonjugat nach Überwindung der Diffusionsbarriereschicht (13) in die elektrodennahe Reaktionsschicht (16) gelangt, an den dort immobiliserten Rezeptor oder Liganden gebunden wird, und die Marker-Phenoloxidase nach der Mobilisierung des Phenoloxidase-Substrats in der Substratschicht (18) und seines Durchdringens der Diffusionsbarriereschicht (17) in die elektrodennahe Reaktionsschicht (16) hinein eine Amplifizierungsreaktion zwischen Phenoloxidase-Substrat und modifizierter Elektrodenoberfläche (3) katalysiert, die ein analytproportionales voltammetrisches Stromsignal liefert.

23. Assay nach Anspruch 19,
**dadurch gekennzeichnet,**
**daß** der zu bestimmende Analyt in der Probeflüssigkeit den Hydrolase-markierten Rezeptor oder Liganden in Schicht (12) mobilisiert, eine pseudohomogene Bindungsreaktion zwischen dem Analyten und dem diffusionsfähigen Hydrolase-markierten Rezeptor oder Liganden erfolgt, der nichtgebundene Anteil des Rezeptor-Konjugats oder Ligand-Konjugats nach Überwindung der Diffusionsbarriereschicht (13) in Schicht (14) mit immobilisiertem Liganden oder Rezeptor gebunden wird, und der Analyt-Rezeptorkonjugat-Komplex oder Analyt-Ligandkonjugat-Komplex nach Überwindung der Diffusionsbarriereschicht (15) in die elektrodennahe Reaktionsschicht (16) gelangt, an einen immobilisierten Rezeptor oder Liganden gebunden wird und die Marker-Hydrolase nach der Mobilisierung des Hydrolase-Substrates in der Substratschicht (20) und seines Durchdringens der Diffusionsbarriereschicht (17) in die elektrodennahe Reaktionsschicht (16) hinein ein Edukt hydrolisiert, das in die zusätzlich vor der Elektrodenoberfläche (3) angeordnete Schicht (21), die immobilisierte Phenoloxidase enthält, eindringt, und als Substrat der Phenoloxidase eine von der Phenoloxidase katalysierte Amplifizierungsreaktion zwischen Phenoloxidase-Substrat und modifizierter Elektrodenoberfläche (3) auslöst, die ein analytproportionales voltammetrisches Stromsignal liefert.

24. Assay nach Anspruch 19,
**dadurch gekennzeichnet,**
**daß** der zu bestimmende Analyt in der Probeflüssigkeit den Hydrolase-markierten Rezeptor oder Liganden in Schicht (12) mobilisiert, Analyt und Hydrolasemarkierter Ligand oder Rezeptor nach Überwindung der Diffusionsbarriereschicht (13) in Schicht (14) mit immobilisiertem Liganden oder immobilisiertem Rezeptor um deren Bindungsstellen konkurriert, und der nichtgebundene Anteil an Hydrolase-markiertem Liganden oder Hydrolase-markiertem Rezeptor nach Überwindung der Diffusionsbarriereschicht (15) in die elektrodennahe Reaktionsschicht (16) gelangt, an den immobilisierten Rezeptor oder Liganden in Schicht (16) gebunden wird, und die Marker-Hydrolase nach der Mobilisierung des Hydrolasesubstrat in der Substratschicht (20) und seines Durchdringens der Diffusionsbarriereschicht (17) in die elektrodennahe Reaktionsschicht (16) hinein ein Edukt hydrolisiert, das in die zusätzlich vor der Elektrodenoberfläche (3) angeordnete Schicht (21), die immobilisierte Phenoloxidase enthält, eindringt, und als Phenoloxidase-Substrat eine von der Phenoloxidase katalysierte Amplifizierungsreaktion zwischen Phenoloxidase-Substrat und modifizierter Elektrodenoberfläche (3) auslöst, die ein analytproportionales voltammetrisches Stromsignal liefert.

25. Assay nach Anspruch 19,
**dadurch gekennzeichnet,**
**daß** der zu bestimmende Analyt in der Probeflüssigkeit die Schicht (19) erreicht, die immobilisierten Liganden oder Rezeptor enthält, deren Bindungsstellen mit Hydrolase-Rezeptorkonjugat oder Hydrolase-Ligandkonjugat, respektive, abgesättigt sind, der Analyt einen Teil des Hydrolase-Ligandkonjugats oder des Hydrolase-Rezeptorkonjugats verdrängt und das mit dem Analyten komplexierte Hydrolase-Ligangkonjugat oder das mit dem Analyten komplexierte Hydrolase-Rezeptorkonjugat nach Überwindung der Diffusionsbarriereschicht (13) in die elektrodennahe Reaktionsschicht (16) gelangt, an den dort immobilisierten Rezeptor oder immobilisierten Liganden gebunden wird, und die Marker-Hydrolase nach der Mobilisierung des Hydrolase-Substrats in der Substratschicht (20) und seines Durchdringens der Diffusionsbarriereschicht (17) in die elektrodennahe Reaktionsschicht (16) hinein ein Edukt hydrolisiert, das in die zusätzlich vor der Elektrodenoberfläche (3) angeordnete Schicht (21), die immobilisierte Phenoloxidase enthält, eindringt, und als Phenoloxidase-Substrat eine von der Phenoloxidase katalysierte Amplifizierungsreaktion zwischen Phenoloxidase-Substrat und modifizierter Elektrodenoberfläche (3) auslöst, die ein analytproportionales voltammetrisches Stromsignal liefert.

26. Verwendung einer Phenoloxidase, vorzugsweise Tyrosinase, als Markerenzym für die affinen Bindungspartner in einem elektrochemischen Affinitätsassay.

27. Verwendung eines phenolische Verbindungen hydrolisierenden Enzyms in Kombination mit einer Phenoloxidase in einem elektrochemischen Affinitätsassay, wobei das hydrolisierende Enzym als Markerenzym für die affinen Bindungspartner eingesetzt wird und die Phenoloxidase als Katalysator für die Amplifizierungsreaktion zwischen Phenoloxidase-Substrat und Redoxmediator dient.

28. Verwendung nach Anspruch 27,
**dadurch gekennzeichnet,**
**daß** als hydrolisierendes Enzym eine Phosphatase oder Galactosidase und als Phenoloxidase Tyrosinase eingesetzt werden.

## Claims

1. An enzymatic-electrochemical single-step affinity sensor for the quantitative determination of analytes in aqueous media, consisting of a support (1) having applied thereon a measuring system (22) or two adjacent measuring systems (22 and 23) of identical design and the contact paths (2) thereof, each measuring system consisting of multiple consecutive layers arranged over a redox electrode (3) modified with an electron mediator, and its pseudo-reference electrode (4), which layers are laterally encapsulated in a liquid-proof fashion by a fixing frame (6), the top cover (8) of which has an area which has openings to receive the sample to be measured, wherein
a) for that case where the marker enzyme of the affine binding partner is a phenol oxidase, the layers comprise a layer (18) including a phenol oxidase substrate, or
b) for that case where the marker enzyme of the affine binding partner is a hydrolase, the layers comprise a layer (20) including a hydrolase substrate, and an additional layer (21) in the vicinity of the electrode, which includes immobilized phenol oxidase,
in addition to a sample-receiving and reservoir layer (11), a layer (16) in the vicinity of the electrode, the layers (12) and (14) which contain affine binding partners, or a layer (19) including appropriate immobilized affinity complexes.

2. The sensor according to claim 1, **characterized in that** in the event of two measuring systems (22, 23), one measuring system represents the actual indication system, and the other measuring system is used to control calibration and function.

3. The sensor according to claim 1 or 2, **characterized in that** the redox electrode, which preferably is a mediator-modified carbon electrode, has a polarization voltage of between -300 mV and 100 mV versus the pseudo-reference electrode which preferably is an Ag/AgCl electrode.

4. The sensor according to any of claims 1 to 3, **characterized in that** the electron mediator of the modified redox electrode is fixed on the electrode surface by adsorption, physical occlusion, covalent bonding, or in the form of a redox polymer.

5. The sensor according to any of claims 1 to 4, **characterized in that** the electron mediator is a quinoid redox dye, a quinone, a redox-active complex compound of iron, ruthenium or tungsten, a metallocene, phthalocyanine, or an electrically conductive redox polymer such as polyaniline, polypyrrole, poly-o-phenyienediamine, or polyacetylene.

6. The sensor according to any of claims 1 to 5, **characterized in that** below the top cover (8) of the fixing frame (6), a water-impermeable film (9) overlapping the perforations of said cover is arranged, which is spaced apart from cover (8) by a spacer (10), and that the film (9) is followed by the sample-receiving and reservoir layer (11).

7. The sensor according to any of claims 1 to 6, **characterized in that** following the sample-receiving and reservoir layer (11), a layer (12) is arranged which either contains a freely diffusible phenol oxidase-labelled ligand or a freely diffusible phenol oxidase-labelled receptor, followed by a diffusion barrier layer (13) and thereafter, a layer (14) having an immobilized ligand or an immobilized receptor is arranged, followed by a second diffusion barrier layer (15) which is followed by the reaction layer (16) in the vicinity of the electrode as the last layer, to which either the ligand or receptor is immobilized, and which is enclosed by an enzyme substrate layer (18) which is separated by a diffusion barrier (17) and contains a substrate for the phenol oxidase.

8. The sensor according to any of claims 1 to 6, **characterized in that** following the sample-receiving and reservoir layer (11), a layer (19) is arranged which either contains an immobilized receptor complexed with a phenol oxidase-labelled ligand, or an immobilized ligand complexed with a phenol oxidase-labelled receptor, followed by a diffusion barrier layer (13) which is followed by the reaction layer (16) in the vicinity of the electrode as the last layer, to which either the ligand or receptor is immobilized, and which is enclosed by an enzyme substrate layer (18) which is separated by a diffusion barrier (17) and contains a substrate for the phenol oxidase.

9. The sensor according to any of claims 1 to 6, **characterized in that** following the sample-receiving and reservoir layer (11), a layer (12) is arranged which either contains a freely diffusible hydrolase-labelled ligand or a freely diffusible hydrolase-labelled receptor, followed by a diffusion barrier layer (13) and thereafter, a layer (14) having an immobilized ligand or an immobilized receptor is arranged, followed by a second diffusion barrier layer (15) which is followed by the reaction layer (16) in the vicinity of the electrode, to which either the ligand or receptor is immobilized, and which is enclosed by an enzyme substrate layer (20) which is separated by a diffusion barrier (17) and contains a substrate for the hydrolase, and an additional layer (21) in the vicinity of the electrode is arranged between layer (16) and redox electrode (3), which layer contains immobilized phenol oxidase.

10. The sensor according to any of claims 1 to 6, **characterized in that** following the sample-receiving and reservoir layer (11), a layer (19) is arranged which either contains an immobilized receptor complexed with a hydrolase-labelled ligand, or an immobilized ligand complexed with a hydrolase-labelled receptor, followed by a diffusion barrier layer (13) which is followed by the reaction layer (16) in the vicinity of the electrode, to which either the ligand or receptor is immobilized, and which is enclosed by an enzyme substrate layer (20) which is separated by a diffusion barrier (17) and contains a substrate for the hydrolase, and an additional layer (21) in the vicinity of the electrode is arranged between layer (16) and redox electrode (3), which layer contains immobilized phenol oxidase.

11. The sensor according to any of claims 1 to 10, **characterized in that** the water-impermeable film (9) is a membrane preferably made of PTFE, polyethylene, polycarbonate, or rubber compounds.

12. The sensor according to any of claims 1 to 11, **characterized in that** the sample-receiving and reservoir layer (11) is capable of swelling and contains a natural or synthetic hydrogel, preferably made of agar, gelatin, pectin, dextrin, polyacrylate, or polyurethane.

13. The sensor according to any of claims 1 to 12, **characterized in that** a hydrophobized paper is used as diffusion barrier layer (13, 15 or 17).

14. The sensor according to any of claims 1 to 13, **characterized in that** the layers (12, 14 or 16) containing the affine binding partners, the layers (18 or 20) containing the enzyme substrate, the phenol oxidase layer (21), or the layer (19) containing the enzyme-labelled, immobilized affinity complexes consist of an absorbent material containing a hydrophilic component, preferably a cellulose, a polysilicate, a linear-cross-linked hydrogel, or a mixture of said materials.

15. The sensor according to claim 14, **characterized in that** a polysaccharide, a polyalcohol, a poly(ether alcohol), an inorganic salt, or a mixture of said components is used as hydrophilic component.

16. The sensor according to any of claims 1 to 15, **characterized in that** tyrosinase is used as phenol oxidase.

17. The sensor according to any of claims 1 to 16, **characterized in that** phenol, m-cresol, p-cresol, 2,4-xylenol, p-chlorophenol, or catechol is used as enzyme substrate of tyrosinase.

18. The sensor according to any of claims 1 to 6 or 9 to 16, **characterized in that** an alkaline phosphatase, acid phosphatase, or (β-galactosidase is used as hydrolyzing enzyme.

19. An enzymatic-electrochemical single-step affinity assay for the quantitative determination of analytes in aqueous media, **characterized in that** the sample fluid is applied to the perforated area of the fixing frame cover (8) of a sensor according to claims 1 to 18 connected to a potentiostat or a manual measuring instrument of potentiostatic design and initially, diffuses into the sample-receiving and reservoir layer (11) via spacer (10), said layer undergoing swelling, thereby pressing the film (9) against the fixing frame cover (8), thus closing same, and subsequently, the analyte to be determined in the sample fluid undergoes an affine binding reaction when passing the layers of the sensor according to claims 1 to 18, wherein
a) a phenol oxidase-labelled receptor, a phenol oxidase-labelled ligand, or the corresponding phenol oxidase-labelled affinity complex, together with the analyte, diffuses to the mediator-modified electrode surface (3), and the phenol oxidase oxidizes a suitable phenol oxidase substrate in layer (18) in immediate vicinity of the electrode surface to form an electrically active product which is reduced cathodically via the reversibly reduced electron mediator to form a starting substrate of the phenol oxidase, or
b) the phenol oxidase on the redox electrode (3) is fixed as a layer (21), and an educt resulting from the reaction of a hydrolyzing enzyme used as label in the same fashion as in a) with a suitable hydrolase substrate in layer (20) is oxidized in the immediate vicinity of the electrode surface by the phenol oxidase used as catalytic layer (21) to form an electrically active product which is reduced cathodically via the reversibly reduced electron mediator to form a starting substrate of the phenol oxidase, the cyclic substrate regeneration generated in both cases a) or b) resulting in a chemically amplified, analyte-proportional cathodic current which is quantified using *per se* common voltammetric methods.

20. The assay according to claim 19, **characterized in that** the analyte to be determined mobilizes the phenol oxidase-labelled ligand or receptor in layer (12), a pseudo-homogeneous binding reaction takes place between the analyte and the diffusible phenol oxidase-labelled receptor or the phenol oxidase-labelled ligand, the non-bound fraction of the receptor or ligand conjugate, subsequent to overcoming the diffusion barrier layer (13), is bound to immobilized ligand or immobilized receptor in layer (14), and the analyte-receptor conjugate complex or the analyte-ligand conjugate complex, subsequent to overcoming the diffusion barrier layer (15), enters the reaction layer (16) in the vicinity of the electrode, being bound to an immobilized receptor or ligand, and the marker phenol oxidase, after mobilization of the phenol oxidase substrate in the substrate layer (18) and its breaking through the diffusion barrier layer (17) into the reaction-layer (16) in the vicinity of the electrode, catalyzes an amplifying reaction between the phenol oxidase substrate and the modified electrode surface (3), thus providing an analyte-proportional voltammetric current signal.

21. The assay according to claim 19, **characterized in that** the analyte to be determined in the sample fluid mobilizes the phenol oxidase-labelled ligand or receptor in layer (12), the analyte and the phenol oxidase-labelled ligand or the phenol oxidase-labelled receptor, subsequent to overcoming the diffusion barrier layer (13), compete with immobilized ligand or receptor for the available binding sites in layer (14), and the non-bound phenol oxidase-labelled ligand or phenol oxidase-labelled receptor, subsequent to overcoming the next diffusion barrier layer (15), enters the reaction layer (16) in the vicinity of the electrode, being bound to an immobilized receptor or ligand, and the marker phenol oxidase, after mobilization of the phenol oxidase substrate in the substrate layer (18) and its breaking through the diffusion barrier layer (17) into the reaction layer (16) in the vicinity of the electrode, catalyzes an amplifying reaction between the phenol oxidase substrate and the modified electrode surface (3), thus providing an analyte-proportional voltammetric current signal.

22. The assay according to claim 19, **characterized in that** the analyte to be determined in the sample fluid reaches layer (19) which contains immobilized ligand or receptor, the binding sites thereof being saturated with phenol oxidase-receptor conjugate or phenol oxidase-ligand conjugate, the analyte displaces part of the phenol oxidase-ligand conjugate or the phenol oxidase-receptor conjugate, and the phenol oxidase-ligand conjugate complexed with analyte or the phenol oxidase-receptor conjugate complexed with analyte, subsequent to overcoming the diffusion barrier layer (13), enters the reaction layer (16) in the vicinity of the electrode, being bound to the receptor or ligand immobilized therein, and the marker phenol oxidase, after mobilization of the phenol oxidase substrate in the substrate layer (18) and its breaking through the diffusion barrier layer (17) into the reaction layer (16) in the vicinity of the electrode, catalyzes an amplifying reaction between the phenol oxidase substrate and the modified electrode surface (3), thus providing an analyte-proportional voltammetric current signal.

23. The assay according to claim 19, **characterized in that** the analyte to be determined in the sample fluid mobilizes the hydrolase-labelled receptor or ligand in layer (12), a pseudo-homogeneous binding reaction between the analyte and the diffusible hydrolase-labelled receptor or ligand takes place, the non-bound fraction of the receptor conjugate or ligand conjugate, subsequent to overcoming the diffusion barrier layer (13), is bound to immobilized ligand or receptor in layer (14), and the analyte-receptor conjugate complex or the analyte-ligand conjugate complex, subsequent to overcoming the diffusion barrier layer (15), enters the reaction layer (16) in the vicinity of the electrode, being bound to an immobilized receptor or ligand, and the marker hydrolase, after mobilization of the hydrolase substrate in the substrate layer (20) and its breaking through the diffusion barrier layer (17) into the reaction layer (16) in the vicinity of the electrode, hydrolyzes an educt which penetrates layer (21) additionally arranged in front of the electrode surface (3) containing immobilized phenol oxidase and, being a substrate of phenol oxidase, triggers a phenol oxidase-catalyzed amplifying reaction between the phenol oxidase substrate and the modified electrode surface (3), which provides an analyte-proportional voltammetric current signal.

24. The assay according to claim 19, **characterized in that** the analyte to be determined in the sample fluid mobilizes the hydrolase-labelled receptor or ligand in layer (12), the analyte and the hydrolase-labelled ligand or receptor, subsequent to overcoming the diffusion barrier layer (13), compete with immobilized ligand or immobilized receptor for the binding sites in layer (14), and the non-bound fraction of hydrolase-labelled ligand or hydrolase-labelled receptor, subsequent to overcoming the diffusion barrier layer (15), enters the reaction layer (16) in the vicinity of the electrode, being bound to an immobilized receptor or ligand in layer (16), and the marker hydrolase, after mobilization of the hydrolase substrate in the substrate layer (20) and its breaking through the diffusion barrier layer (17) into the reaction layer (16) in the vicinity of the electrode, hydrolyzes an educt which penetrates layer (21) additionally arranged in front of the electrode surface (3) containing immobilized phenol oxidase and, being a substrate of phenol oxidase, triggers a phenol oxidase-catalyzed amplifying reaction between the phenol oxidase substrate and the modified electrode surface (3), which provides an analyte-proportional voltammetric current signal.

25. The assay according to claim 19, **characterized in that** the analyte to be determined in the sample fluid reaches layer (19) which contains immobilized ligand or receptor, the binding sites thereof being saturated with hydrolase-receptor conjugate or hydrolase-ligand conjugate, the analyte displaces part of the hydrolase-ligand conjugate or the hydrolase-receptor conjugate, and the hydrolase-ligand conjugate complexed with analyte or the hydrolase-receptor conjugate complexed with analyte, subsequent to overcoming the diffusion barrier layer (13), enters the reaction layer (16) in the vicinity of the electrode, being bound to the receptor or ligand immobilized therein, and the marker hydrolase, after mobilization of the hydrolase substrate in the substrate layer (20) and its breaking through the diffusion barrier layer (17) into the reaction layer (16) in the vicinity of the electrode, hydrolyzes an educt which penetrates layer (21) additionally arranged in front of the electrode surface (3) containing immobilized phenol oxidase and, being a substrate of phenol oxidase, triggers a phenol oxidase-catalyzed amplifying reaction between the phenol oxidase substrate and the modified electrode surface (3), which provides an analyte-proportional voltammetric current signal.

26. Use of a phenol oxidase, preferably tyrosinase, as marker enzyme for the affine binding partners in an electrochemical affinity assay.

27. Use of an enzyme hydrolyzing phenolic compunds in combination with a phenol oxidase in an electrochemical affinity assay, the hydrolyzing enzyme being employed as marker enzyme for the affine binding partners, and the phenol oxidase being used as catalyst for the amplifying reaction between the phenol oxidase substrate and the redox mediator.

28. The use according to claim 27, **characterized in that** a phosphatase or galactosidase is employed as hydrolyzing enzyme, and tyrosinase is employed as phenol oxidase.

## Revendications

1. Détecteur d'affinité à un seul cycle de traitement enzymatico-électrochimique pour la détermination quantitative des analytes en milieu aqueux, comportant un support (1), sur lequel est disposé un système de mesure (22) ou deux systèmes de mesure identiques situés l'un à côté de l'autre (22,23) et leur circuit de contact (2), dans lequel chaque système de mesure est composé de plusieurs couches disposées les unes à la suite des autres au dessus d'une électrode redox (3) modifiée avec un médiateur d'électrons et de son électrode de pseudoréférence (4), qui sont capsulées de chaque côté de manière à être étanche aux fluides à l'aide d'un cadre de fixation (6), et dont le recouvrement supérieur (8) offre une surface qui possède des ouvertures pour la réception de l'échantillon à mesurer, dans lequel les couches contiennent soit
a) dans le cas où l'enzyme marqueur du partenaire de liaison affine est de la phénoloxydase, une couche (18) contenant un substrat de phénoloxydase, soit
b) dans le cas où l'enzyme marqueur du partenaire de liaison affine est une hydrolase, une couche (20) contenant un substrat d'hydrolase et une couche additionnelle (21) proche de l'électrode comportant de la phénoloxydase immobilisée,
en plus d'une couche réservoir (11) d'absorption de l'échantillon, d'une couche (16) proche de l'électrode, des couches (12) et (14) qui contiennent des partenaires de liaison affines, ou d'une couche (19) qui contient des complexes d'affinité immobilisés correspondants.

2. Détecteur selon la revendication 1,
**caractérisé en ce**
**que** dans le cas des deux systèmes de mesure (22,23), l'un des systèmes de mesure représente le système d'indication proprement dit et l'autre système de mesure sert au contrôle d'étalonnage et au contrôle fonctionnel.

3. Détecteur selon la revendication 1 ou 2,
**caractérisé en ce que**
l'électrode redox, qui est de préférence une électrode de carbone modifiée par un médiateur, présente, vis à vis de l'électrode de pseudoréférence qui est de préférence une électrode Ag/AgCl, une tension de polarisation comprise entre -300 mV et 100 mV.

4. Détecteur selon une des revendications de 1 à 3,
**caractérisé en ce que**
le médiateur d'électrons de l'électrode modifiée est fixé sur la surface des électrodes par adsorption, par inclusion physique, par liaison covalente ou sous forme d'un polymère redox.

5. Détecteur selon une des revendications de 1 à 4,
**caractérisé en ce que**
le médiateur d'électrons est un colorant redox quinoïde, une quinone, des composés complexes redox-actifs du fer, du ruthénium ou du tungstène, un métallocène, de la phthalocyanine ou un polymère électriquement conducteur comme la polyaniline, le polypyrrole, le poly-o-phénylènediamine ou le polyacétylène.

6. Détecteur selon une des revendications de 1 à 5,
**caractérisé en ce**
**qu'**une feuille (9) imperméable à l'eau chevauchant les perforations du recouvrement est appliquée sous le recouvrement supérieur (8) du cadre de fixation (6), cette feuille étant maintenue à distance du recouvrement (8) par un espaceur (10), et que la couche réservoir de réception de l'échantillon (11) est rattachée à la suite de cette feuille (9).

7. Détecteur selon une des revendications de 1 à 6,
**caractérisé en ce**
**qu'**à la suite de la couche réservoir de réception d'échantillon (11), est disposée une couche (12) contenant un ligand diffusant marqué à la phénoloxydase ou un récepteur diffusant marqué à la phénoloxydase, suivie par une couche de barrière de diffusion (13), puis par une couche (14) comportant un ligand immobilisé ou un récepteur immobilisé, une seconde couche de barrière de diffusion (15), et ensuite, comme dernière couche, par la couche de réaction (16) proche de l'électrode, sur laquelle le ligand ou le récepteur est immobilisé, qui est enveloppée par une couche (18) de substrat pour un enzyme contenant un substrat pour la phénoloxydase, séparée par une barrière de diffusion (17).

8. Détecteur selon une des revendications de 1 à 6,
**caractérisé en ce**
**qu'**à la suite de la couche réservoir de réception d'échantillon (11), est disposée une couche (19) contenant un récepteur immobilisé qui est complexé avec un ligand marqué à la phénoloxydase ou un ligand immobilisé qui est complexé avec un récepteur marqué à la phénoloxydase, suivie par une couche de barrière de diffusion (13), puis, en tant que dernière couche, la couche de réaction (16) proche de l'électrode, sur laquelle est immobilisé un ligand ou un récepteur, et qui est entourée par une couche (18) de substrat pour l'enzyme, séparée par une barrière de diffusion (17), contenant un substrat pour la phénoloxydase.

9. Détecteur selon une des revendications de 1 à 6,
**caractérisé en ce**
**qu'**à la suite de la couche réservoir de réception d'échantillon (11), est disposée une couche (12) contenant un ligand diffusant marqué à l'hydrolase ou un récepteur diffusant marqué à l'hydrolase, suivie par une couche de barrière de diffusion (13), puis par une couche (14) comportant un ligand immobilisé ou un récepteur immobilisé, une seconde couche de barrière de diffusion (15), et ensuite, comme dernière couche, par la couche de réaction (16) proche de l'électrode, sur laquelle le ligand ou le récepteur est immobilisé, qui est enveloppée par une couche de substrat pour un enzyme (20) contenant un substrat pour l'hydrolase qui est séparée par une barrière de diffusion (17), et en ce qu'une couche supplémentaire (21) proche de l'électrode contenant la phénoloxydase immobilisée est disposée entre la couche (16) et l'électrode redox (3).

10. Détecteur selon une des revendications de 1 à 6,
**caractérisé en ce**
**qu'**à la suite de la couche réservoir de réception d'échantillon (11), est disposée une couche (19) contenant un récepteur immobilisé qui est complexé avec un ligand marqué à l'hydrolase ou un ligand immobilisé qui est complexé avec un récepteur marqué à l'hydrolase, rattachée ensuite à la couche de réaction (16) proche de l'électrode, sur laquelle est immobilisé un ligand ou un récepteur, et qui est enveloppée par une couche (20) de substrat pour l'enzyme, séparée par une barrière de diffusion (17), contenant un substrat pour l'hydrolase, et en ce qu'une couche supplémentaire (21) proche de l'électrode contenant la phénoloxydase immobilisée est disposée entre la couche (16) et l'électrode redox (3).

11. Détecteur selon une des revendications de 1 à 10,
**caractérisé en ce que**
la feuille (9) imperméable à l'eau est une membrane qui est de préférence en PTFE, polyéthylène, polycarbonate ou en composés de caoutchouc.

12. Détecteur selon une des revendications de 1 à 11,
**caractérisé en ce que**
la couche réservoir de réception d'échantillon (11) est gonflable et contient un hydrogel naturel ou synthétique, de préférence d'agar-agar, de gélatine, de pectine, de dextrine, de polyacrylate ou de polyuréthane.

13. Détecteur selon une des revendications de 1 à 12,
**caractérisé en ce**
**qu'**en tant que couche de barrière de diffusion (13,15 ou 17), l'on utilise un papier rendu hydrophobe.

14. Détecteur selon une des revendications de 1 à 13,
**caractérisé en ce**
**que** les couches (12,14 ou 16) contenant les partenaires de liaison affines, les couches (18 ou 20) contenant le substrat de l'enzyme, la couche de phénoloxydase (21) ou la couche (19) contenant les complexes d'affinité immobilisés marqués à l'enzyme sont constituées d'une matière absorbante contenant un composant hydrophile, de préférence d'une cellulose, d'un polysilicate, d'un hydrogel réticulé de façon linéaire ou d'un mélange des matières citées.

15. Détecteur selon la revendication 14,
**caractérisé en ce que**
le composant hydrophile utilisé est un polysaccharide, un polyalcool, un polyéther-alcool, un sel minéral ou un mélange des composants cités.

16. Détecteur selon une des revendications de 1 à 15,
**caractérisé en ce**
**qu'**en tant que phénoloxydase, l'on utilise de la tyrosinase.

17. Détecteur selon une des revendications de 1 à 16,
**caractérisé en ce**
**qu'**en tant que substrat d'enzyme pour la tyrosinase, l'on utilise du phénol, du m-crésol, du p-crésol, du 2,4-xylénol, du p-chlorophénol ou du catéchol.

18. Détecteur selon une des revendications de 1 à 6 ou de 9 à 16,
**caractérisé en ce**
**qu'**en tant qu'enzyme hydrolysante, l'on utilise une phosphatase alcaline, une phosphatase acide ou de la β-galactosidase.

19. Analyse d'affinité à un seul cycle de traitement enzymatico-électrochimique pour la détermination quantitative des analytes en milieu aqueux,
**caractérisé en ce que**
le fluide de l'échantillon est appliqué sur la surface perforée du recouvrement du cadre de fixation (8) d'un détecteur selon les revendications 1 à 18, qui est relié à un potentiostat ou à un instrument de mesure manuel comportant un potentiostat, et que ce liquide diffuse d'abord via l'espaceur (10) dans la couche réservoir de réception de l'échantillon (11), qu'il gonfle cette couche et comprime la feuille (9) sur le recouvrement du cadre de fixation (8), l'obture et qu'ensuite, l'analyte à déterminer dans le fluide de l'échantillon produit une réaction de liaison affine par passage à travers les couches du détecteur selon les revendications 1 à 18 de la manière suivante :
a) diffusion d'un récepteur marqué à la phénoloxydase, d'un ligand marqué à la phénoloxydase ou du complexe d'affinité marqué à la phénoloxydase correspondant avec l'analyte à la surface (3) des électrodes modifiée par un médiateur et oxydation par la phénoloxydase d'un substrat de la phénoloxydase approprié dans la couche (18) à proximité immédiate de la surfaces des électrodes en un produit électroactif qui est réduit par un médiateur d'électrons réduit de manière réversible par voie cathodique en un substrat initial de la phénoloxydase, ou
b) fixation de la phénoloxydase sur l'électrode redox (3) en tant que couche (21), obtention d'un éduit par réaction d'une enzyme hydrolysante utilisée comme marqueur de la même manière que dans a) avec un substrat d'hydrolase approprié dans la couche (20), oxydation de cet éduit par la phénoloxydase utilisée comme couche catalytique (21) à proximité immédiate de la surface des électrodes en un produit électroactif, qui est réduit par un médiateur d'électrons réduit de manière réversible par voie cathodique en un substrat initial de la phénoloxydase,
dans lequel, dans les deux cas a) ou b), la régénération cyclique du substrat produite génère un courant cathodique amplifié chimiquement proportionnel à l'analyte qui est quantifié par des méthodes voltamétriques habituelles.

20. Analyse selon la revendication 19,
**caractérisé en ce que**
l'analyte à déterminer mobilise le ligand ou le récepteur marqué à la phénoloxydase dans la couche (12), qu'il se produit une réaction de liaison pseudohomogène entre l'analyte et le récepteur marqué à la phénoloxydase ou le ligand marqué à la phénoloxydase aptes à diffuser, que la proportion non liée du conjugué de récepteur ou de ligand se lie après avoir traversé la couche barrière de diffusion (13) avec un ligand immobilisé ou un récepteur immobilisé dans la couche (14), que le complexe formé analyte-conjugué du récepteur ou le complexe analyte-conjugué du ligand parvient à la couche de réaction (16) proche de l'électrode après avoir traversé la couche barrière de diffusion (15), qu'un de ces complexes se lie à un ligand ou un récepteur immobilisé, et que le marqueur phénoloxydase, après la mobilisation du substrat de phénoloxydase dans la couche de substrat (18) et son passage au travers de la couche barrière de diffusion (17) et dans la couche de réaction (16) proche des électrodes, catalyse une réaction d'amplification entre le substrat de phénoloxydase et la surface des électrodes (3) modifiée, qui délivre un signal de courant voltamétrique proportionnel à l'analyte.

21. Analyse selon la revendication 19,
**caractérisé en ce que**
l'analyte à déterminer dans le fluide de l'échantillon mobilise le ligand marqué à la phénoloxydase ou le récepteur marqué à la phénoloxydase dans la couche (12), l'analyte et le ligand marqué à la phénoloxydase ou le récepteur marqué à la phénoloxydase, après avoir traversé la couche barrière de diffusion (13), entrent en compétition dans la couche (14) avec le ligand ou le récepteur immobilisé autour des sites de liaison actifs, le ligand marqué à la phénoloxydase non combiné ou le récepteur marqué à la phénoloxydase non combiné, après avoir traversé la couche barrière de diffusion (15), parvient dans la couche de réaction (16) proche de l'électrode, puis se lie au récepteur ou au ligand immobilisé dans la couche (16), et **en ce que** le marqueur phénoloxydase, après la mobilisation du substrat de phénoloxydase dans la couche de substrat (18) et son passage au travers de la couche barrière de diffusion (17) et dans la couche de réaction (16) proche des électrodes, catalyse une réaction d'amplification entre le substrat de phénoloxydase et la surface des électrodes (3) modifiée, qui délivre un signal de courant voltamétrique proportionnel à l'analyte.

22. Analyse selon la revendication 19,
**caractérisé en ce que**
l'analyte à déterminer dans le fluide de l'échantillon atteint la couche (19) contenant le ligand ou le récepteur immobilisé, dont les sites de liaison avec le conjugué phénoloxydase-ligand ou le conjugué phénoloxydase-récepteur sont respectivement saturés, l'analyte déplace une partie du conjugué phénoloxydase-ligand ou du conjugué phénoloxydase-récepteur, le conjugué phénoloxydase-ligand complexé avec l'analyte ou le conjugué phénoloxydase-récepteur complexé avec l'analyte parvient, après avoir traversé la couche barrière (13), jusqu'à la couche de réaction proche des électrodes (16), il se combine avec le ligand ou le récepteur qui y est immobilisé et **en ce que** le marqueur phénoloxydase, après la mobilisation du substrat de phénoloxydase dans la couche de substrat (18) et son passage au travers de la couche barrière de diffusion (17) et dans la couche de réaction (16) proche des électrodes, catalyse une réaction d'amplification entre le substrat de phénoloxydase et la surface des électrodes (3) modifiée, en délivrant un signal de courant voltamétrique proportionnel à l'analyte.

23. Analyse selon la revendication 19,
**caractérisé en ce que**
l'analyte à déterminer dans le fluide de l'échantillon mobilise le ligand ou le récepteur marqué à l'hydrolase dans la couche (12), qu'il se produit une réaction de liaison pseudohomogène entre l'analyte et le récepteur marqué à l'hydrolase ou le ligand marqué à l'hydrolase aptes à diffuser, que la proportion non liée du conjugué de récepteur ou du conjugué de ligand se lie après avoir traversé la couche barrière de diffusion (13) avec un ligand immobilisé ou un récepteur immobilisé dans la couche (14), que le complexe formé analyte-conjugué du récepteur ou le complexe analyte-conjugué du ligand parvient jusqu'à la couche de réaction (16) proche de l'électrode après avoir traversé la couche barrière de diffusion (15), qu'un de ces complexes se lie à un ligand ou un récepteur immobilisé, et que le marqueur hydrolase, après la mobilisation du substrat d'hydrolase dans la couche de substrat (20) et son passage au travers de la couche barrière de diffusion (17) et dans la couche de réaction (16) proche des électrodes, hydrolyse un éduit qui pénètre dans la couche (21) disposée en plus avant la surface des électrodes (3), contenant la phénoloxydase immobilisée, qui, en tant que substrat de phénoloxydase, déclenche une réaction d'amplification catalysée par la phénoloxydase entre le substrat de phénoloxydase et la surface des électrodes modifiée (3), qui délivre un signal de courant voltamétrique proportionnel à l'analyte.

24. Analyse selon la revendication 19,
**caractérisé en ce que**
l'analyte à déterminer dans le fluide de l'échantillon mobilise le récepteur ou le ligand marqué à l'hydrolase dans la couche (12), l'analyte et le ligand ou le récepteur marqué à l'hydrolase, après avoir traversé la couche barrière de diffusion (13), entrent en compétition dans la couche (14) avec le ligand ou le récepteur immobilisé autour de leurs sites de liaison, la partie non combinée du ligand marqué à l'hydrolase ou du récepteur marqué à l'hydrolase, après avoir traversé la couche barrière de diffusion (15), parvient dans la couche de réaction (16) proche de l'électrode, puis se lie au récepteur ou au ligand immobilisé dans la couche (16), et **en ce que** le marqueur hydrolase, après la mobilisation du substrat d'hydrolase dans la couche de substrat (20) et son passage au travers de la couche barrière de diffusion (17) et dans la couche de réaction (16) proche des électrodes, hydrolyse un éduit qui pénètre dans la couche (21) disposée en plus avant la surface des électrodes (3), contenant la phénoloxydase immobilisée, et qui, en tant que substrat de phénoloxydase, déclenche une réaction d'amplification catalysée par la phénoloxydase entre le substrat de phénoloxydase et la surface des électrodes modifiée (3), qui délivre un signal de courant voltamétrique proportionnel à l'analyte.

25. Analyse selon la revendication 19,
**caractérisé en ce que**
l'analyte à déterminer dans le fluide de l'échantillon atteint la couche (19) contenant le ligand ou le récepteur immobilisé, dont les sites de liaison avec le conjugué hydrolase-récepteur ou le conjugué hydrolase-ligand sont respectivement saturés, l'analyte déplace une partie du conjugué hydrolase-ligand ou du conjugué hydrolase-récepteur, le conjugué hydrolase-ligand complexé avec l'analyte ou le conjugué hydrolase-récepteur complexé avec l'analyte parvient, après avoir traversé la couche barrière (13), jusqu'à la couche de réaction (16) proche des électrodes, il se combine avec le ligand ou le récepteur qui y est immobilisé et **en ce que** le marqueur hydrolase, après la mobilisation du substrat d'hydrolase dans la couche de substrat (20) et son passage au travers de la couche barrière de diffusion (17) et dans la couche de réaction (16) proche des électrodes, hydrolyse un éduit qui pénètre dans la couche (21) complémentaire disposée avant la surface des électrodes (3) contenant la phénoloxydase immobilisée, et qui, en tant que substrat de phénoloxydase, déclenche une réaction d'amplification catalysée par la phénoloxydase entre le substrat de phénoloxydase et la surface des électrodes modifiée (3), qui délivre un signal de courant voltamétrique proportionnel à l'analyte.

26. Utilisation d'une phénoloxydase, de préférence d'une tyrosinase, en tant qu'enzyme marqueur pour le partenaire de liaison affine dans une analyse d'affinité électrochimique.

27. Utilisation d'un enzyme hydrolysant les composés phénoliques en combinaison avec une phénoloxydase dans une analyse d'affinité électrochimique,
dans lequel l'enzyme hydrolysant est utilisée comme enzyme marqueur pour le partenaire de liaison affine et que la phénoloxydase sert de catalyseur pour la réaction d'amplification entre le substrat de phénoloxydase et le médiateur redox.

28. Utilisation selon la revendication 27,
**caractérisée en ce**
**qu'**en tant qu'enzyme hydrolysant, l'on utilise une phosphatase ou une galactosidase et qu'en tant que phénoloxydase, l'on utilise de la tyrosinase.
